# EUROPEAN PATENT SPECIFICATION

(11) **EP 3 473 706 B1**
(45) Date of publication and mention of the grant of the patent: **10.09.2025**
(21) Application number: 17813289.0
(22) Date of filing: 13.06.2017
(51) Int. Cl.: C12N 5/077, A61K 35/36, A61K 47/42, A61P 17/14

(54) **CELL-EMBEDDED BEADS FOR HAIR REGENERATION AND METHOD FOR PRODUCING SAME, AND KIT FOR HAIR REGENERATION**
IN ZELLEN EINGEBETTETE KÜGELCHEN ZUR HAARREGENERATION UND VERFAHREN ZUR HERSTELLUNG DAVON SOWIE KIT ZUR HAARREGENERATION
BILLES ENCHÂSSÉES DE CELLULES DESTINÉES À LA RÉGÉNÉRESCENCE CAPILLAIRE ET LEUR PROCÉDÉ DE PRODUCTION, ET KIT DE RÉGÉNÉRESCENCE CAPILLAIRE

(30) Priority: 17.06.2016 JP 2016120808
(43) Date of publication of application: 24.04.2019
(73) Proprietor: National University Corporation Yokohama National University, Yokohama-shi, Kanagawa 240-8501 (JP); Kanagawa Institute of Industrial Science and Technology, Ebina-shi Kanagawa 243-0435 (JP)
(72) Inventor: FUKUDA, Junji, Yokohama-shi Kanagawa 240-8501 (JP); KAGEYAMA, Tatsuto, Yokohama-shi Kanagawa 240-8501 (JP)
(74) Representative: Schnappauf, Georg
(86) International application number: PCT/JP2017/021770
(87) International publication number: WO 2017/217393

(56) References cited:
- WO-A1-2017/073625
- JP-A- 2003 070 466
- JP-A- 2017 051 160
- US-A1- 2007 116 680
- US-A1- 2012 027 860
- US-A1- 2016 051 722
- JULIA F. MARKUSEN ET AL: "Behavior of Adult Human Mesenchymal Stem Cells Entrapped in Alginate-GRGDY Beads", TISSUE ENGINEERING, vol. 12, no. 4, 1 April 2006 (2006-04-01), pages 821 - 830, XP055000136, ISSN: 1076-3279, DOI: 10.1089/ten.2006.12.821
- WEI SEONG TOH ET AL: "Advances in hydrogel delivery systems for tissue regeneration", MATERIALS SCIENCE AND ENGINEERING C., vol. 45, 1 December 2014 (2014-12-01), CH, pages 690 - 697, XP055228821, ISSN: 0928-4931, DOI: 10.1016/j.msec.2014.04.026
- ARCHANA BHAT ET AL: "Alginate hydrogels containing cell-interactive beads for bone formation", THE FASEB JOURNAL, vol. 27, no. 12, 1 December 2013 (2013-12-01), US, pages 4844 - 4852, XP055639309, ISSN: 0892-6638, DOI: 10.1096/fj.12-213611
- WILLIAMS C G ET AL: "In vitro chondrogenesis of bone marrow-derived mesenchymal stem cells in a photopolymerizing hydrogel", TISSUE ENGINEERING, LARCHMONT, NY, US, vol. 9, no. 4, 1 August 2003 (2003-08-01), pages 679 - 688, XP002356385, ISSN: 1076-3279, DOI: 10.1089/107632703768247377
- CHISA YOSHIMURA ET AL.: "Mohatsu Saiseino o Yusuru Moho Soshiki no Bio Fabrication = [Biofabrication of hair follicle tissue with hair regenerating ability]", ABSTRACTS OF AUTUMN MEETING OF THE SOCIETY OF CHEMICAL ENGINEERS. SCEJ AUTUMN MEETING ; 47 (SAPPORO) : 2015.09.09-11 , vol. 47, 2015, Japan, pages ZB1P68, XP009514806
- TATSUTO KAGEYAMA ET AL.: "YP3-3 Kan'yokei Saibo Homai Gel Beads o Mochiita Mohatsu Saisei Gijutsu no Kaihatsu = [Development of hair regeneration technology using mesenchymal cell embedded gel beads]", THE JAPANESE JOURNAL OF ARTIFICIAL ORGANS, vol. 45, no. 2, October 2016 (2016-10-01), pages S-155, XP009514293, ISSN: 0300-0818
- TATSUTO KAGEYAMA ET AL.: "Bisai Kako o Mochiita Mohatsu Saisei no Tameno Saibo Baiyozara", PUBLISHTEXTILE PROCESSING TECHNOLOG, vol. 51, no. 11, November 2016 (2016-11-01), pages 620 - 625, XP009518284

## Description

### Technical Field

The present teaching relates to cell-embedded beads for hair regeneration, a method for producing the same, and a kit for hair regeneration.

The present application claims priority on the basis of Japanese Patent Application No. 2016-120808 filed in Japan on June 17, 2016.

### Background Art

In order to establish hair follicle regenerative medicine sufficient for clinical application, a regenerated hair follicle needs to have a normal tissue structure, and hair having a hair shaft suited for a transplantation site needs to be formed and grow. Generally, ectodermal appendages including skin appendages such as hair develop during the fetal period by the interaction between epithelial cells and mesenchymal cells. It is known that a hair follicle, which is one of the ectodermal appendages, experiences growth and regression (hair cycle) that repeats throughout an individual's life, and during the growth period, the regeneration of a hair bulb portion is induced by the same molecular mechanism as that in the developmental period of hair follicle organs. **In** addition, the regeneration of a hair bulb portion in the hair cycle is considered to be induced by a hair papilla cell, which is a mesenchymal cell. Namely, during the growth period, an epithelial stem cell of a hair follicle is induced to differentiate from a hair papilla cell, which is a mesenchymal cell, and a hair bulb portion is regenerated.

**In** the past, for the hair follicle regeneration, the regeneration of hair follicle variable regions by the substitution of mesenchymal cells (hair papilla cells and dermal root sheath cells), the neogenesis of hair follicles using mesenchymal cells having a hair follicle-inducing ability, the reconstruction of hair follicles using epithelial cells and mesenchymal cells, and the like have been tried. More specifically, for example, a method of culturing a cell mixture using a culture solution obtained by adding a Wnt signal activator to a plurality of kinds of somatic cells so as to form primordial hair follicle organs (for example, see PTL 1), a method of preparing artificial hair bulbs in which epithelial cells adhere to the exterior of a cell cluster (spheroid) of hair follicle mesenchymal cells (for example, see PTL 2), a method for regenerating hair by transplanting spheroids of hair papilla cells together with epithelial cells (for example, see NPL 1) and the like have been tried.

### Citation List

### Patent Literature

[PTL 1] Japanese Unexamined Patent Application, First Publication No. 2013-78344
[PTL 2] Japanese Unexamined Patent Application, First Publication No. 2003-70466

### Non-Patent Literature

[NPL 1] Huang, Y-C., et al., "Scalable production of controllable dermal papilla spheroids on PVA surfaces and the effects of spheroid size on hair follicle regeneration", Biomaterials, Vol. 34, p. 442-451, 2013

### Summary of the Invention

The present invention is defined by the appended claims. The present disclosure provides teachings which in some respects go beyond the disclosure of the invention as such, which is defined exclusively by the appended claims. The teachings are provided to place the actual invention in a broader technical context and to illustrate possible related technical developments. Such additional technical information which does not fall within the scope of the appended claims is not part of the invention. In particular, the term "embodiment" is not to be construed as necessarily referring to an embodiment of the invention, unless the "embodiment" in question falls within the scope of the claims.

### Technical Problem

In conventional methods for regenerating hair follicles, although methods by which normal hair is regenerated from a transplanted portion by transplanting regenerated hair follicle organs to skin in vitro were typical, there were problems with hair regeneration efficiency.

With the foregoing in view, the present teaching provides cell-embedded beads for hair regeneration having high hair regeneration activity and a method for producing the same.

### Solution to Problem

**As a result** of conducting extensive studies to solve the aforementioned problem, the inventors of the present teaching found that cell-embedded beads for hair regeneration prepared using mesenchymal cells have high hair regeneration activity, thereby leading to completion of the present teaching.

The present teaching includes the following aspects.

The cell-embedded beads for hair regeneration according to a first aspect of the present teaching contain mesenchymal cells and a biocompatible hydrogel.

The biocompatible hydrogel may be an extracellular matrix component which gelates.

The extracellular matrix component may also be type I collagen.

The cell density of the mesenchymal cells may be 2 × 10⁵ cells/cm³ or less.

The method for producing cell-embedded beads for hair regeneration according to a second aspect of the present teaching is provided with a cell-embedded cured gel formed body preparation step for preparing a cell-embedded cured gel formed body by preparing liquid droplets containing mesenchymal cells and a biocompatible hydrogel and curing the biocompatible hydrogel, and an aggregation step for suspension-culturing the cell-embedded cured hydrogel formed body and aggregating using the tractive force of the cells.

In the cell-embedded cured gel formed body preparation step, the liquid droplets may be prepared by dropping onto a support having a water-repellent surface.

In the cell-embedded cured gel formed body preparation step, the concentration of the mesenchymal cells contained in the liquid droplets may be 5 × 10⁵ cells/mL to 1 × 10⁸ cells/mL.

The kit for hair regeneration according to a third aspect of the present teaching is provided with the cell-embedded beads for hair regeneration according to the first aspect and epithelial cells.

### Advantageous Effects of Teaching

According to the present teaching, cell-embedded beads for hair regeneration having high hair regeneration activity and a method for producing the same can be provided.

### Brief Description of Drawings

FIG 1 is a process diagram schematically illustrating the method for producing cell-embedded beads for hair regeneration according to a first embodiment of the present teaching
FIG 2(A) shows images illustrating cell-embedded beads for hair regeneration after three days of culturing in Example 1 and the results of observing spheroids using a phase-contrast microscope (IX-71, Olympus). FIG 2(B) shows images of cell-embedded beads for hair regeneration after three days of culturing in Example 1 and the results of HE staining of spheroids.
FIG 3 shows images of cell-embedded beads for hair regeneration after three days of culturing in Example 1 and the results of alkaline phosphatase (ALP) staining of a monolayer culture.
FIG 4A is a graph indicating the results of quantifying alkaline phosphatase (ALP) activity of cell-embedded beads for hair regeneration after three days of culturing, spheroids and a monolayer culture according to the Bessey-Lowry method in Test Example 1.
FIG 4B is a graph indicating the cell number of cell-embedded beads for hair regeneration after three days of culturing in, spheroids and a monolayer culture in Test Example 1.
FIG 5 is a graph indicating the relative gene expression of Versican relative to the expression of GAPDH in cell-embedded beads for hair regeneration after three days of culturing and spheroids in Test Example 2.
FIG 6A is a graph indicating the relative expression of Igfbp5 relative to the expression of GAPDH in cell-embedded beads for hair regeneration after three days of culturing and spheroids in Test Example 3.
FIG 6B is a graph indicating the relative expression of Tgfb2 relative to the expression of GAPDH in cell-embedded beads for hair regeneration after three days of culturing and spheroids in Test Example 3.
FIG 7 is a diagram schematically illustrating a method for subcutaneously transplanting a mixture of cell-embedded beads for hair regeneration or spheroids after three days of culturing and epithelial cells to a nude mouse according to the patch method in Test Example 4.

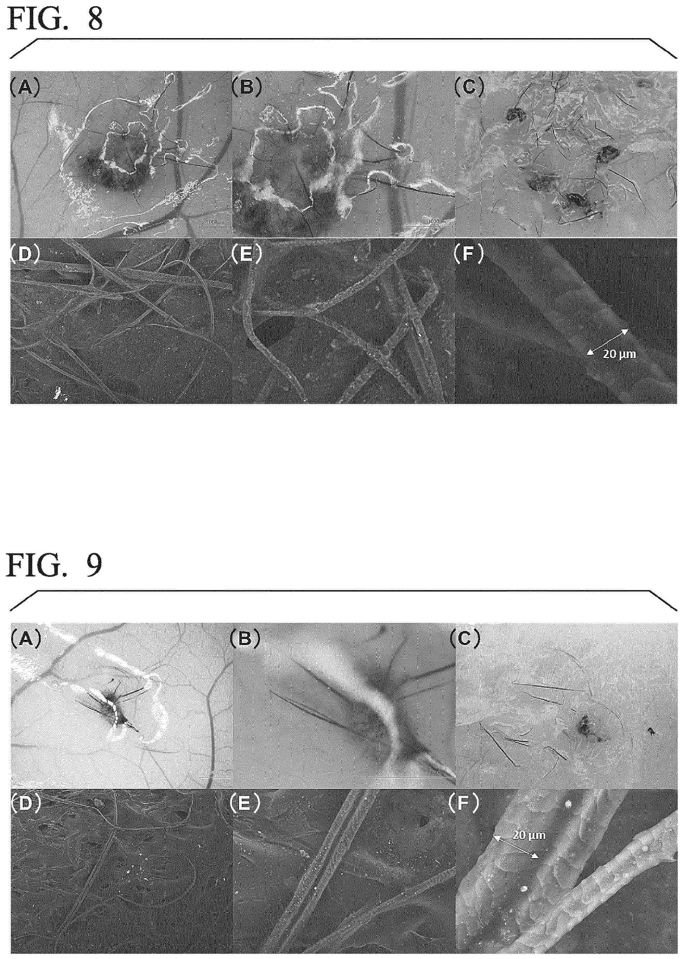

FIGS. 8(A) and 8(B) show images of regenerated hair at transplantation sites of a nude mouse one month after transplanting a mixture of cell-embedded beads for hair regeneration of Example 1 and epithelial cells as photographed using a digital microscope (VHX-1000, Keyence) in Test Example 4. FIG 8(C) shows the results of having extracted hair of the interior portion of FIG 8(A) in Test Example 4 as photographed using a digital microscope (VHX-1000, Keyence). FIGS. 8(D) to 8(F) show images of regenerated hair at transplantation sites of a nude mouse one month after transplanting a mixture of cell-embedded beads for hair regeneration of Example 1 and epithelial cells as photographed using a scanning electron microscope (SEM) in Test Example 4.
FIGS. 9(A) and 9(B) show images of regenerated hair at transplantation sites of a nude mouse one month after transplanting a mixture of mesenchymal cells of Comparative Example 2 and epithelial cells as photographed using a digital microscope (VHX-1000, Keyence) in Test Example 4. FIG 9(C) shows the results of having extracted hair of the interior portion of FIG 9(A) in Test Example 4 as photographed using a digital microscope (VHX-1000, Keyence). FIGS. 9(D) to 9(F) show images of regenerated hair at transplantation sites of a nude mouse one month after transplanting a mixture of spheroids of Comparative Example 2 and epithelial cells as photographed using a scanning electron microscope (SEM) in Test Example 4.
FIG 10A is a graph indicating the generated hair number at transplantation sites of a nude mouse one month after transplanting cell-embedded beads for hair regeneration of Example 1 or spheroids of mesenchymal cells of Comparative Example 2 in Test Example 4.
FIG 10B is a graph indicating the thickness of regenerated hair at transplantation sites of a nude mouse one month after transplanting cell-embedded beads for hair regeneration of Example 1 or spheroids of mesenchymal cells of Comparative Example 2 in Test Example 4.
FIG 11A shows images indicating the results of observing cell-embedded beads for hair regeneration (cell number of mesenchymal cells: 5 × 10³ cells/bead, 10 × 10³ cells/bead, 20 × 10³ cells/bead or 40 × 10³ cells/bead) after three days of culturing in Example 2 using a phase-contrast microscope (IX-71, Olympus).
FIG 11B is a graph indicating changes over time in the diameter of cell-embedded beads for hair regeneration (cell number of mesenchymal cells: 5 × 10³ cells/bead, 10 × 10³ cells/bead, 20 × 10³ cells/bead or 40 × 10³ cells/bead) in Example 2.

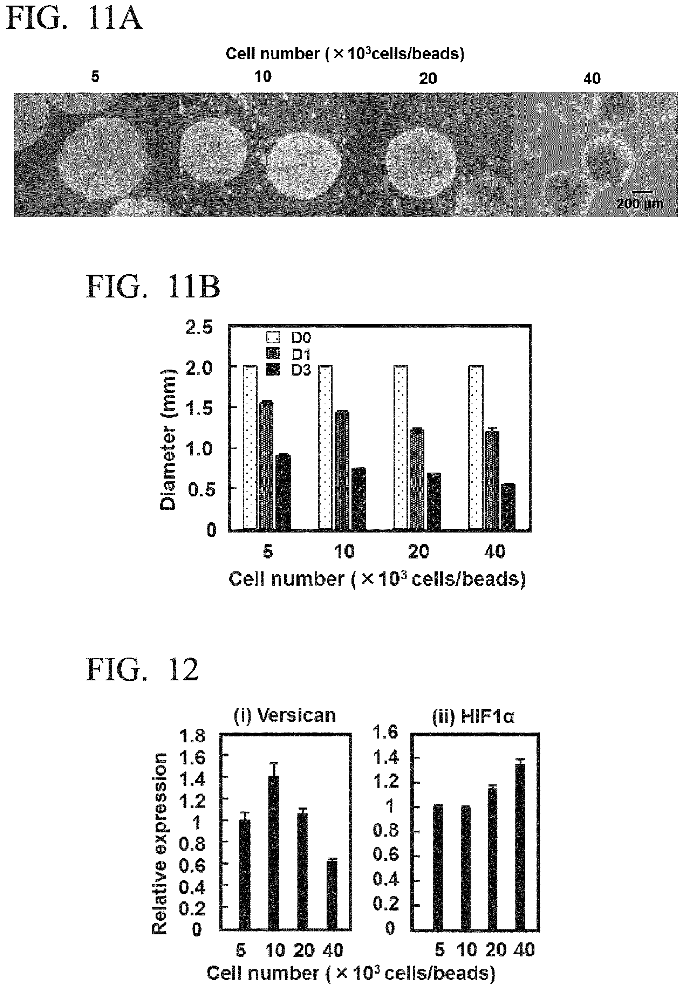

FIG 12(i) is a graph indicating the relative expression of Versican relative to the expression of GAPDH in cell-embedded beads for hair regeneration (cell number of mesenchymal cells: 5 × 10³ cells/bead, 10 × 10³ cells/bead, 20 × 10³ cells/bead or 40 × 10³ cells/bead) after three days of culturing of Example 2 in Test Example 5. FIG 12(ii) is a graph indicating the relative expression of HIF1α relative to the expression of GAPDH in cell-embedded beads for hair regeneration (cell number of mesenchymal cells: 5 × 10³ cells/bead, 10 × 10³ cells/bead, 20 × 10³ cells/bead or 40 × 10³ cells/bead) after three days of culturing of Example 2 in Test Example 5.
FIG 13A shows images indicating the results of observing cell-embedded beads for hair regeneration (collagen concentration: 0.75 mg/mL, 1.5 mg/mL, 2.25 mg/mL or 3.0 mg/mL) after three days of culturing in Example 3 using a phase-contrast microscope (IX-71, Olympus).
FIG 13B is a graph indicating changes over time in the diameter of cell-embedded beads for hair regeneration (collagen concentration: 0.75 mg/mL, 1.5 mg/mL, 2.25 mg/mL or 3.0 mg/mL) in Example 3.
FIG 14 is a graph indicating the relative expression of Versican relative to the expression of GAPDH in cell-embedded beads for hair regeneration (collagen concentration: 0.75 mg/mL, 1.5 mg/mL, 2.25 mg/mL or 3.0 mg/mL) after three days of culturing of Example 3 in Test Example 6.

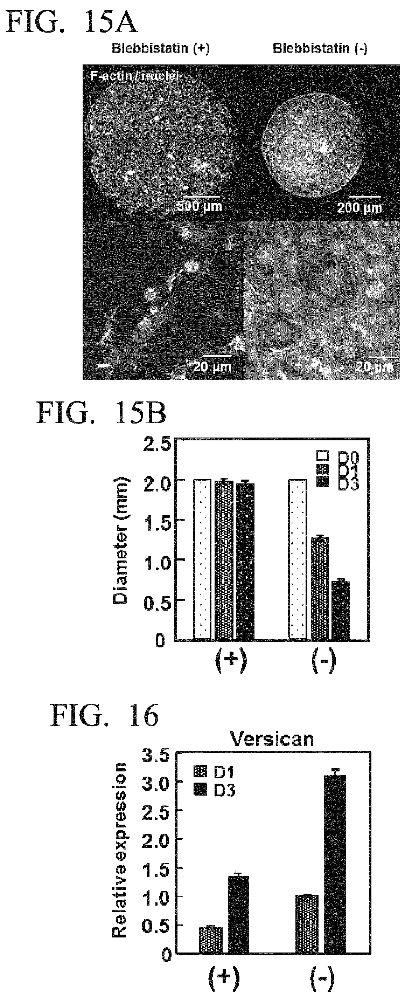

FIG 15A (upper row) shows images indicating the results of observing cell-embedded beads for hair regeneration after three days of culturing in the absence of addition (-) of blebbistatin of Example 4 and cell-embedded beads for hair regeneration after three days of culturing in the presence of addition (+) of blebbistatin of Comparative Example 3 using a confocal laser microscope (LSM700, Carl Zeiss). FIG 15A (lower row) shows images indicating the results of observing cell-embedded **beads for** hair regeneration after three days of culturing in the absence of addition (-) of blebbistatin of Example 4 and cell-embedded beads for hair regeneration after three days of culturing in the presence of addition (+) of blebbistatin of Comparative Example 3 using a confocal laser microscope (LSM700, Carl Zeiss).
FIG 15B is a graph indicating changes over time in the diameter of cell-embedded beads for hair regeneration after three days of culturing in the absence of addition (-) of blebbistatin of Example 4 and cell-embedded beads for hair regeneration after three days of culturing in the presence of the addition (+) of blebbistatin of Comparative Example 3.
FIG 16 is a graph indicating the relative expression of Versican relative to the expression of GAPDH in cell-embedded beads for hair regeneration on day 1 and day 3 from the start of culturing in the absence of the addition (-) of blebbistatin of Example 4 and cell-embedded beads for hair regeneration on day 1 and day 3 from the start of culturing in the presence of the addition (+) of blebbistatin of Comparative Example 3 in Test Example 7.
FIG 17A is a diagram schematically illustrating a method for subcutaneously transplanting a mixture of cell-embedded beads for hair regeneration of Example 5 and epithelial cells (hair beads: HB) or hair follicle germs (HFG) of Reference Example 1 into a nude mouse according to the patch method in Test Example 8.
FIG 17B(i) shows an image of regenerated hair at the transplantation site of a nude mouse three weeks after transplantation of the HB of Example 5 as photographed using a digital microscope (VHX-1000 Keyence) in Test Example 8. FIG 17(ii) shows an enlarged image of FIG 17(i). FIG 17(iii) shows an image of regenerated hair at a transplantation site of a nude mouse three weeks after transplantation of the HFG of Reference Example 1 as photographed using a digital microscope (VHX-1000, Keyence). FIG 17(iv) is enlarged image of FIG 17(iii).
FIG 17C is a graph indicating the generated hair number at transplantation sites of a nude mouse three weeks after transplantation of the HB of Example 5 and the HFG of Reference Example 1 in Test Example 8.
FIG 18A a) shows an image of regenerated hair at a transplantation site of a nude mouse three weeks after transplantation of mixture the cell-embedded beads for hair regeneration prepared using human papilla cells of Example 6 (HB2) and epithelial cells as photographed using a digital microscope (VHX-1000, Keyence) in Test Example 9. FIG 18A b) shows an image of regenerated hair at a transplantation site of a nude mouse three weeks after transplantation of hair follicle germs of Reference Example 2 (HFG2) as photograph using a digital microscope (VHX-1000, Keyence) in Test Example 9.
**FIG** 18B is a graph indicating the generated hair number at a transplantation site of a nude mouse three weeks after transplantation of the HB2 of Example 6 or the HFG2 of Reference Example 2 in Test Example 9.
FIG 18C shows an image indicating the results of HE staining of a skin section of a transplantation site of a nude mouse three weeks after transplantation of the HB2 of Example 6 in Test Example 9.
**FIG** 18D shows an image of regenerated hair at a transplantation site of a nude mouse three weeks after transplant of the HB2 of Example 6 as photographed using a SEM in Text Example 9.

### Description of Embodiments

Hereinafter, embodiments of the present teaching will be specifically explained with reference to the drawings as necessary.

### <<Cell-Embedded Beads for Hair Regeneration>>

The cell-embedded beads for hair regeneration according to one embodiment of the present teaching contain mesenchymal cells and biocompatible hydrogel.

The cell-embedded beads for hair regeneration of the present embodiment have high hair regeneration activity. Consequently, use of the cell-embedded beads for hair regeneration of the present embodiment makes it possible to realize high hair regeneration efficiency.

In the present description, "mesenchymal cells" refer to cells derived from mesenchymal tissues or obtained by culturing such cells. Specific examples of mesenchymal cells include hair papilla cells, dermal root sheath cells, skin mesenchymal cells in a developmental period and hair follicle mesenchymal cells induced from pluripotent cells (for example, embryonic stem (ES) cells, embryonic germ (EG) cells, and induced pluripotent stem (iPS) cells).

In the present description, "epithelial cells" refer to cells derived from epithelial tissues or cells obtained by culturing such cells. Specific examples thereof include cells of the outermost layer of the outer root sheath in the bulge region, epithelial cells of the hair matrix portion, hair follicle epithelial cells induced from pluripotent cells (for example, embryonic stem (ES) cells, embryonic germ (EG) cells, and induced pluripotent stem (iPS) cells).

These cells are preferably derived from animals, more preferably derived from vertebrates, and particularly preferably derived from humans.

In the present description, "cell-embedded beads for hair regeneration" refer to beads containing mesenchymal cells and biocompatible hydrogen as previously described that are aggregated by the tractive force of cells and can be used as is for biological transplant in the production method to be subsequently described.

In the present description, "hair follicle primordium" refers to a tissue which is the rudiment of a hair follicle, and is constituted with the aforementioned mesenchymal cells and the aforementioned epithelial cells. The flow of the formation of hair follicle primordium is such that, first, the epithelial cells thicken and invaginate toward the mesenchymal cell side to engulf a cell cluster (spheroid) of the mesenchymal cells. Next, the epithelial cells engulfing the spheroid of the mesenchymal cells form a hair matrix primordium, and the spheroid of the mesenchymal cells forms a hair papilla primordium composed of the hair matrix primordium and the hair papilla. In the hair follicle primordium, hair papilla cells provide growth factors to the hair matrix cells and induce the differentiation of the hair matrix cells, and the differentiated cells are able to form hair.

In the present description, "hair follicle" refers to a skin appendage that produces hair and is a portion where the epidermis is depressed inwardly in the form of a cylinder.

In the present description, "regenerated hair follicle primordium" refers to, for example, hair follicle primordium regenerated using the cell-embedded beads for hair regeneration of the present embodiment and epithelial cells.

In addition, in the present description, "hair regeneration activity" refers to an increase in the expression of a gene involved in regeneration of hair in mesenchymal cells. Examples of this gene include Versican, Wnt, transforming growth factor-β (TGF-β) and fibroblast growth factor (FGF).

In the present embodiment, the cell density of the aforementioned mesenchymal cells is required to be dense, and is 2 × 10⁵ cells/cm³, preferably 5 × 10⁴ cells/cm³ to 2 × 10⁵ cells/cm³, more preferably 7.5 × 10⁴ cells/cm³ to 1.5 × 10⁵ cells/cm³, even more preferably 9 × 10⁴ cells/cm³ to 1.2 × 10⁵ cells/cm³ and particular preferably 1 × 10⁵ cells/cm³. By making cell density to be within the aforementioned ranges, cell density approaches that of hair papilla and it is possible to realize high hair regeneration efficiency.

**In** the present description, "biocompatible hydrogel" refers to a gel having compatibility with the body that is a substance in which polymers adopt a network structure by chemical bonding and retain a large amount of water in the network. More specifically, the biocompatible hydrogel refers to a substance obtained by introducing crosslinks into an artificial material of a polymer derived from a natural substance or a synthetic polymer and gelating the cross-linked substance.

Examples of the polymer derived from a natural substance include an extracellular matrix component which gelates. Examples of the extracellular matrix component which gelates include collagen (such as type I, type II, type III, type V or type XI), a basal membrane component (trade name: Matrigel) reconstructed from a mouse EHS tumor extract (including type IV collagen, laminin, heparan sulfate proteoglycan and the like), fibrin, glycosaminoglycan, hyaluronic acid and proteoglycan. Gelatin, agar or agarose and the like can be used as other polymers derived from natural substances. The hydrogel can be prepared by selecting components such as salts optimal for gelation as well as the concentration or pH and the like thereof. **In** addition, these raw materials may also be combined.

Examples of the synthetic polymer include polyacrylamide, polyvinyl alcohol, methyl cellulose, polyethylene oxide and poly(II-hydroxyethylmethacrylate)/polycaprolactone. The hydrogel can also be prepared using two or more types of these polymers.

Among these, the biocompatible hydrogel is preferably a polymer derived from a natural substance, more preferably an extracellular matrix component that gelates, and even more preferably that containing type I collagen. As a result of containing type I collagen, the composition of the biocompatible hydrogel approaches that of hair papilla and it is possible to realize high hair regeneration efficiency.

### <<Method for Producing Cell-Embedded Beads for Hair Regeneration>>

The method for producing cell-embedded beads for hair regeneration according to one embodiment of the present teaching is a method provided with a cell-embedded cured gel formed body preparation step for preparing a cell-embedded cured gel formed body by preparing liquid droplets containing mesenchymal cells and a biocompatible hydrogel and curing the biocompatible hydrogel, and an aggregation step for suspension-culturing the cell-embedded cured hydrogel formed body and aggregating using the tractive force of the cells.

According to the production method of the present embodiment, cell-embedded beads for hair regeneration can be obtained that have high hair regeneration activity.

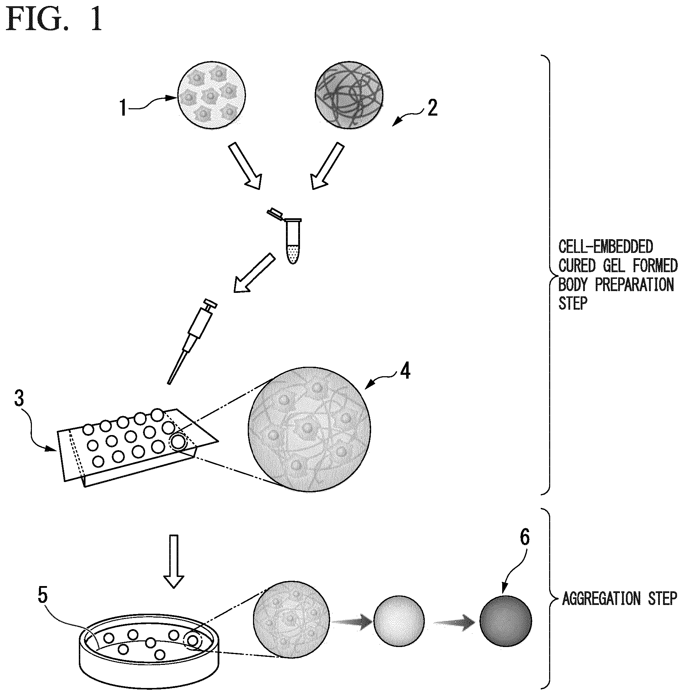

FIG 1 is a process diagram schematically illustrating the method for producing cell-embedded beads for hair regeneration according to a first embodiment of the present teaching. The following provides a detailed explanation of the method for producing cell-embedded beads for hair regeneration of the present embodiment with reference to FIG. 1.

### [Cell-Embedded Cured Gel Formed Body Preparation Step]

First, drops of a size corresponding to the purpose of use are prepared by suspending mesenchymal cells 1 in a solution containing biocompatible hydrogel 2.

The mesenchymal cells used in the production method of the present embodiment are as previously described.

The cells are preferably derived from animals, more preferably from vertebrates and particularly preferably from humans.

**In** the production method of the present embodiment, examples of biocompatible hydrogen are the same as those previously described. Among these, the biocompatible hydrogel is preferably a polymer derived from a natural substance, more preferably an extracellular matrix component that gelates, and even more preferably type I collagen. As a result of using type I collagen, the composition of the biocompatible hydrogel approaches that of hair papilla and it is possible to realize high hair regeneration efficiency.

The solution containing the biocompatible hydrogel may also contain a serum-free medium such as Ham's Nutrient Mixture F-10 or Ham's Nutrient Mixture F-12 or a buffer for reconstructing a biocompatible hydrogel (such as a buffer solution composed of sodium hydroxide, sodium hydrogen carbonate or HEPES buffer).

**In** the case the biocompatible hydrogel is collagen, the concentration of collagen in the solution is, for example, 1.0 mg/mL to 10 mg/mL or for example, 2.0 mg/mL to 5.0 mg/mL. As a result of making the collagen concentration in the solution to be within the aforementioned ranges, expression of Versican gene, which is a marker for hair growth (hair follicle induction), increases and hair can be regenerated with higher efficiency.

The concentration of the aforementioned mesenchymal cells contained in the suspension containing mesenchymal cells and biocompatible hydrogen as previously described is preferably 5.0 × 10⁵ cells/mL to 1.0 × 10⁸ cells/mL, more preferably 2.5 × 10⁶ cells/mL to 5.0 × 10⁷ cells/mL, and even more preferably 2.5 × 10⁶ cells/mL to 2.5 × 10⁷ cells/mL. As a result of making the concentration of the mesenchymal cells to be within the aforementioned ranges, cell density after the aggregation step to be subsequently described approaches the cell density of hair papilla and high hair regeneration efficiency can be realized.

There are not particular limitations on the method used to prepare the droplets and can be determined by a person with ordinary skill in the art in accordance with known methods. Examples thereof include a method for preparing droplets by dropping the aforementioned suspension containing mesenchymal cells and biocompatible hydrogel into a solution composed of an oily component, and a method for preparing droplets by dropping the aforementioned suspension into a support 3 having a water-repellent surface as in FIG 1 and the examples to be subsequently described.

**In** the present description, "water-repellent" refers to the property of repelling water. More specifically, water-repellent typically refers to a water contact angle of 90° or more, preferably 100° or more, and even more preferably 105° or more. **In** addition, water-repellent includes a super-water-repellent state demonstrating a water contact angle in excess of 150°.

A support having a water-repellent surface can be obtained by laminating a material subjected to water-repellent treatment or having water repellency on the surface of the support.

There are no particular limitations on the water-repellent treatment and examples thereof include a method in which a low polarity functional group such as an alkyl group, alkenyl group, alkoxy group or alkenyloxy group, or a hydrophobic functional group such as an alkyl group or alkoxy group in which one or more hydrogen atoms are substituted with fluorine atoms, is introduced onto a substrate surface. In addition, there are no particular limitations on the material having water repellency and examples thereof include hydrophobic polymers such as polypropylene, polyethylene or polysulfone, and chlorine- or fluorine-containing polymers such as polyvinylidene chloride, polyvinylidene fluoride or polytetrafluoroethylene.

There are no particular limitations on the material of the support and examples thereof include, but are not limited to, resin (such as polyester resin, polystyrene resin, polyethylene resin, polypropylene resin, ABS (acrylonitrile-butadiene-styrene) resin, nylon resin, acrylic resin, fluororesin, polycarbonate resin, polyolefin resin, polyurethane resin, polyvinylidene chloride, methylpentene resin, phenol resin, melamine resin, PEEK resin, epoxy resin or vinyl resin), metal (such as gold, silver, copper, aluminum, tungsten, molybdenum, chromium, platinum, titanium or nickel), alloy (such as stainless steel, Hastelloy, Inconel, Monel or Duralumin), glass (such as glass, quartz glass, fused quartz, alumina, sapphire, ceramics, forsterite or photosensitive glass), semiconductor materials, silicon and rubber (such as natural or synthetic rubber). In addition, a plurality of materials may be combined as these materials.

Next, the biocompatible gel is cured by allowing the prepared droplets to stand undisturbed for 20 minutes to not longer than 60 minutes (and preferably, 30 minutes) at 25°C to not higher than 40°C (and preferably, 37°C). Continuing, the surface is washed with a basal medium such as Dulbecco's Modified Eagle's Medium (DMEM) followed by recovery of the cell-embedded cured gel formed body.

### [Aggregation Step]

Next, the aforementioned recovered cell-embedded cured gel formed body is suspension-cultured for 1 day to 10 days and preferably 2 days to 4 days at 25°C to not higher than 40°C (and preferably 37°C).

There are no particular limitations on the medium used and is a basal medium containing components required for cell survival and growth (such as inorganic salts, carbohydrates, hormones, essential amino acids, non-essential amino acids and vitamins). Examples thereof include, but are not limited to, DMEM, Minimum Essential Medium (MEM), RPMI-1640, Basal Medium Eagle (BME), Dulbecco's Modified Eagle's Medium: Nutrient Mixture F-12 (DMEM/F-12) and Glasgow Minimum Essential Medium (Glasgow MEM).

As a result of suspension-culturing the cell-embedded cured gel formed body 4 under the aforementioned conditions, cell-embedded beads for hair regeneration 6 having a cell density similar to that exemplified for the previously described cell-embedded beads for hair regeneration can be obtained by aggregating using the tractive force of cells.

In addition, in the aggregation step in the present embodiment, a series of developmental processes consisting of aggregation of mesenchymal cells and the formation of hair papilla, which are actually carried out in skin tissue of the body, can be reproduced. Consequently, the cell-embedded beads for hair regeneration 6 having high hair activity can be obtained.

### <<Kit for Hair Regeneration>>

The kit for hair regeneration according to one embodiment of the present teaching is provided with the aforementioned cell-embedded beads for hair regeneration and epithelial cells.

According to the kit for hair regeneration of the present embodiment, hair can be regenerated with high efficiency.

Examples of the cell-embedded beads for hair regeneration and the epithelial cells include the same as those exemplified for the aforementioned cell-embedded beads for hair regeneration.

The mesenchymal cells contained in the cell-embedded beads for hair regeneration and the epithelial cells are preferably derived from animals, more preferably from vertebrates, and particularly preferably from humans.

The kit for hair regeneration of the present embodiment may also be provided with a support able to be used to immobilize the cell-embedded beads for hair regeneration during transplant to be subsequently described.

**In** addition, the kit for hair regeneration of the present embodiment may also be further provided with instruments (such as tweezers) used to transplant the cell-embedded beads for hair regeneration and epithelial cells.

### <<Method for Using Cell-Embedded Beads for Hair Regeneration>>

### <Method for Transplanting Cell-Embedded Beads for Hair Regeneration>

During transplant, the cell-embedded beads for hair regeneration of the present embodiment and the epithelial cells are preferably transplanted to a transplantation site simultaneously.

Examples of epithelial cells used for transplant include the same as those exemplified for the aforementioned cell-embedded beads for hair regeneration.

In addition, the ratio of mesenchymal cells contained in the cell-embedded beads for hair regeneration of the present embodiment to epithelial cells is preferably 0.1 times to 100 times and more preferably 1 time to 10 times.

In addition, as indicated in the examples to be subsequently described, in the previously described method for producing the cell-embedded beads for hair regeneration, the epithelial cells and cell-embedded cured gel formed body may be mixed after the cell-embedded cured gel formed body preparation step, and a mixture of cell-embedded beads for hair regeneration and epithelial cells may be prepared by going through the aggregation step. Hair follicle primordia can be regenerated and hair can be regenerated by transplanting the resulting mixture of cell-embedded beads for hair regeneration and epithelial cells to a transplantation site.

The cell-embedded beads for hair regeneration of the present embodiment can be transplanted to a target site using a method known among persons with ordinary skill in the art. For example, the cell-embedded beads for hair regeneration can be transplanted by using a Shapiro's hair transplant procedure, a hair transplant procedure using a Choi's hair transplanter or an implanter using air pressure. The Shapiro's hair transplant procedure is a method consisting of making an incision for transplantation at a transplantation site using a micro-scalpel and the like and performing the transplantation procedure using tweezers.

The transplanted amount of the cell-embedded beads for hair regeneration of the present embodiment is suitably adjusted in consideration of the age, gender, symptoms, treatment site or treatment time and the like of the test animal (various species of mammals including humans and non-human animals and preferably humans).

**In** addition, the transplantation depth can be suitably altered depending on the site targeted for regeneration. For example, the transplantation depth may be 0.05 mm to 5 mm, 0.1 mm to 1 mm or 0.3 mm to 0.5 mm. **In** addition, the transplantation site is preferably such that the cell-embedded beads for hair regeneration are transplanted into the dermal layer of the test animal, and more preferably, is located above the interface between the dermis and subcutaneous tissue where hair follicle regeneration and subsequent hair growth efficiency are superior. In addition, if the transplantation depth is adjusted such that the upper end portions of the epithelial cell components of the hair follicle primordia are exposed through the upper end portion of the incision for transplantation, continuity with the hair follicle cells of the test animal can be further enhanced, thereby making this preferable.

The cell-embedded beads for hair regeneration of the present embodiment may be immobilized at the target transplantation site using a tape or band for skin j oining or sutures and the like.

In addition, the cell-embedded beads for hair regeneration of the present embodiment may also be immobilized using a support. There are no particular limitations on the material of the support provided the material is able to facilitate the connection between the portion on the epithelial cell side of the formed hair follicle primordia and the epithelial cell of the test animal after transplantation. Specific examples of the support include fibers formed of a polymer such as nylon or a synthetic or natural bioabsorbable polymer, metal fiber such as stainless steel, carbon fiber, chemical fiber such as glass fiber, natural animal fiber (hair derived from a biological body) and natural plant fiber, and more specifically, examples of the support include nylon thread and stainless steel wire. The diameter and length of the support can be suitably designed according to the portion targeted for regeneration. For example, the diameter may be 5 µm to or 100 µm or 20 µm to 50 µm. In addition, for example, the length may be 1 mm to 10 mm or 4 mm to 6 mm.

In addition, in the cell-embedded beads for hair regeneration of the present embodiment, in the case of using a support, shortly after the cell-embedded beads for hair regeneration of the present embodiment and the epithelial cells have been transplanted, the support can be removed from the transplantation site after having secured continuity between the epithelial cells of the test animal and the side of the regenerated hair follicle primordia on the derived from the epithelial cells. Although the time at which the support is removed can be suitably set according to the conditions after transplantation, the support is preferably removed from the transplantation site from 3 days to 7 days after transplantation. Alternatively, the support can also be allowed to remain until it is spontaneously removed from the transplantation site. The support made of a bioabsorbable material can be allowed to remain until it is removed spontaneously, decomposed or absorbed.

In the cell-embedded beads for hair regeneration of the present embodiment, in the case of using a support, cells derived from the epithelial cells of the hair follicle primordia grow along the support. As a result, continuity between the epithelial cells of the test animal having undergone transplantation and the epithelial cells of the hair follicle primordia can be improved. In particular, in the case the support is maintained on the exterior of the epidermis of the transplantation portion, since the epithelial cells of the test animal grow toward the interior of the transplantation portion along the support such that foreign substances are excluded, continuity can be further improved. Moreover, hair follicles can be promoted to be formed in an intended direction. As a result, it is possible to improve the rate of hair growth from the hair follicle primordia and to control the direction of hair growth.

### <Method for Regenerating Hair>

In addition, one aspect of the present teaching provides a pharmaceutical composition for treating defective hair sites such as an epidermis defect or hair loss caused by a disease or accident and the like.

In addition, one aspect of the present teaching provides a pharmaceutical composition containing a therapeutically effective amount of cell-embedded beads for hair regeneration.

In addition, one aspect of the present teaching provides a therapeutic agent for hair follicle regeneration containing the aforementioned pharmaceutical composition.

In addition, one aspect of the present teaching provides a use of the aforementioned cell-embedded beads for hair regeneration in order to produce a therapeutic agent for hair regeneration containing the aforementioned pharmaceutical composition.

In addition, one aspect of the present teaching provides a method for treating a defective hair site such as an epidermis defect or hair loss caused by a disease or accident that includes transplanting an effective amount of the aforementioned cell-embedded beads for hair regeneration to a patient requiring treatment.

There are no particular limitations on tissue including hair follicle tissue that is able to be regenerated provided it is the epidermis of a body for which regeneration of hair and hair follicles is desired, and an example thereof is the scalp.

In addition, examples of applicable diseases include, but are not limited to, any arbitrary disease that is accompanied by hair loss such as Androgenetic Alopecia (AGA), Female Androgenetic Alopecia (FAGA), postpartum alopecia, diffuse alopecia, alopecia seborrheica, alopecia pityroides, traction alopecia, metabolic error-induced alopecia, pressure alopecia, alopecia areata, alopecia neurotica, trichotillomania, alopecia totalis and alopecia symptomatica.

There are no particular limitations on the object of treatment, and examples thereof include mammals including humans and non-humans, with humans being preferable.

### Examples

Although the following provides a more detailed description of the present teaching based on examples thereof, the present teaching is not limited to these examples.

### [Example 1] Preparation of Cell-Embedded Beads for Mouse Hair Regeneration 1

### (1) Collection of Mesenchymal Cells and Epithelial Cells

Mesenchymal cells and epithelial cells were collected using a partial modification of the method described in the following reference material: "Toyoshima, K., et al., "Fully functional hair follicle regeneration through the rearrangement of stem cells and their niches", Nat. Commun., Vol. 3, No. 784, 2012". More specifically, skin was collected from the back of an 18-day-old mouse fetus within the uterus of a pregnant C57BL/6jjcl mouse and the skin was treated with Dispase^{®} II (Wako) for 1 hour under conditions of 4°C and 30 rpm followed by separating the epithelial layer and mesenchymal layer. Next, the epithelial layer was treated for 2 hours with collagenase (Wako) at 100 U/mL and for 10 minutes with trypsin to isolate epithelial cells. On the other hand, the mesenchymal layer was treated for 2 hours with collagenase (Wako) at 100 U/mL to isolate mesenchymal cells. The number of resulting mesenchymal cells was 1.0 × 10⁷ cells and the number of epithelial cells was 1.0 × 10⁶ cells.

### (2) Cell-Embedded Cured Gel Formed Body Preparation Step

Next, Dulbecco's Modified Eagle Medium (DMEM, containing 10% fetal bovine serum and 1% penicillin/streptomycin (P/S)) was added to 2.5 × 10⁶ of the collected mesenchymal cells followed by removing the supernatant after centrifuging for 180 seconds at 1000 rpm. Next, the mesenchymal cells were suspended in 1 mL of collagen type 1 (containing 3 mg/mL of collagen, Nitta Gelatin).

The suspension was dropped 2 µL at a time (5.0 × 10³ cells/2 µL) onto the back of a culture dish cover. The suspension was then incubated for 30 minutes at 37°C to prepare the cell-embedded cured gel formed body.

### (3) Aggregation Step

Next, the cell-embedded cured gel formed product was detached from the back of the culture dish cover using Pipetman. Next, the cell-embedded cured gel formed body was placed in a culture dish followed by the addition of DMEM (containing 10% FBS and 1% P/S) and suspension-culturing for 3 days to prepare cell-embedded beads for hair regeneration.

### (4) Observation Results

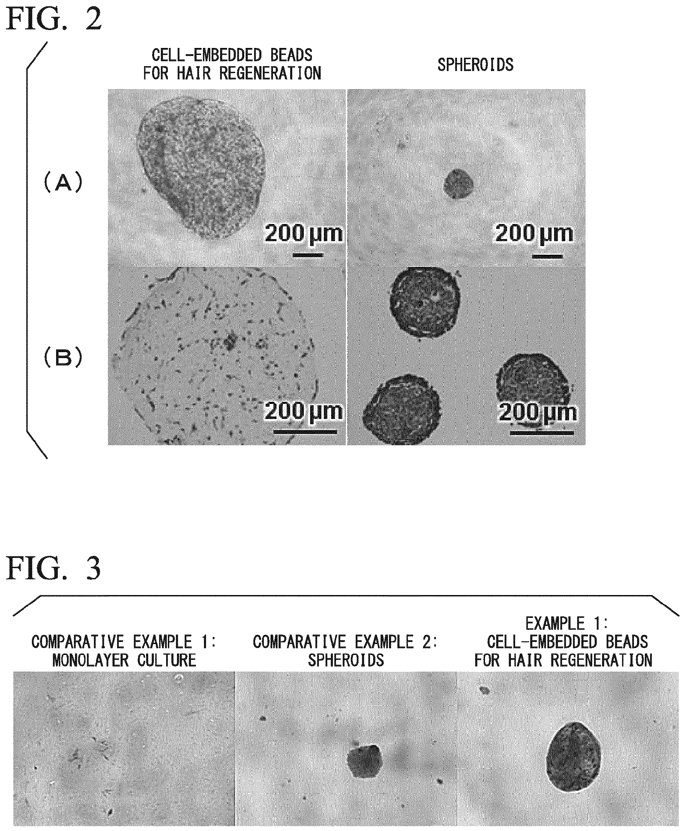

The results of observing the cell-embedded beads for hair regeneration three days after the start of culturing using a phase-contrast microscope (IX-71, Olympus) are shown in FIG 2(A).

Based on FIG 2(A), the cell-embedded beads for hair regeneration were confirmed to aggregate from a mean diameter of 2 mm to 0.5 mm due to the tractive force of the cells.

### (5) Hematoxylin-Eosin (HE) Staining of Cell-Embedded Beads for Hair Regeneration

### (5-1) Preparation of Sections of Cell-Embedded Beads for Hair Regeneration

The cell-embedded beads for hair regeneration were washed with phosphate-buffered saline (PBS) and then fixed with 4% para-formaldehyde. Next, the fixative was recovered followed by immersing for 1 hour each in 70% ethanol, 90% ethanol, 100% ethanol and 2-butanol. Next, the cell-embedded beads for hair regeneration were sliced to an extremely thin thickness using a microtome. The thin sections were transferred to a slide glass by directly pressing in the perpendicular direction to the slide glass.

### (5-2) Hematoxylin-Eosin (HE) Staining

1 mL of xylene were dropped onto the resulting slide glass and allowed to stand undisturbed for 1 hour followed by removing the solvent. Next, 1 mL of 100% ethanol was dropped onto the slide glass and allowed to stand undisturbed for 5 minutes followed by removing the solvent and repeating the procedure one more time. Next, 1 mL of 90% ethanol was dropped onto the slide glass and allowed to stand undisturbed for 5 minutes followed by removing the solvent. Next, 1 mL of 70% ethanol was dropped onto the slide glass and allowed to stand undisturbed for 5 minutes followed by removing the solvent. Next, 1 mL of distilled water was dropped onto the slide glass and allowed to stand undisturbed for 3 minutes followed by removing the water. Next, 1 mL of Mayer's hematoxylin stain (Wako) was dropped onto the slide glass and allowed to stand undisturbed for 4 minutes followed by removing the solvent. Next, the slide glass was rinsed by immersing in running water for 13 minutes. Next, 1 mL of Emajin Solution (Muto Pure Chemicals) was dropped onto the slide glass and allowed to stand undisturbed for 4 minutes followed by removing the solvent. Next, 1 mL of distilled water was dropped onto the slide glass and allowed to stand undisturbed for 1 minute followed by removing the water. Next, 1 mL of 70% ethanol was dropped onto the slide glass and allowed to stand undisturbed for 5 minutes followed by removing the solvent. Next, 1 mL of 90% ethanol was dropped onto the slide glass and allowed to stand undisturbed for 5 minutes followed by removing the solvent. Next, 1 mL of 100% ethanol was dropped onto the slide glass and allowed to stand undisturbed for 5 minutes followed by removing the solvent. Next, 1 mL of 100% ethanol was dropped onto the slide glass followed by repeating the same procedure one more time. Next, 1 mL of xylene was dropped onto the slide glass and allowed to stand undisturbed for 5 minutes followed by removing the solvent. Next, 1 mL of xylene was dropped onto the slide glass followed by repeating the same procedure one more time. Once the slide glass had dried, a small amount of Mount Quick (sealant) was dropped onto the slide glass followed by slowly covering the slide glass with a micro cover glass so as to allow the entry of air bubbles and sealing the contents therein. The results of observing with a phase-contrast microscope (IX-71, Olympus) are shown in FIG 2(B).

### (5-3) Results and Discussion

Based on FIG 2, although cytoplasm is stained pink in the case of staining the cell-embedded beads for hair regeneration with HE stain, portions that were not stained pink were present at several locations. It is surmised that the portions that were not stained are portions where large amounts of collagen are present.

### [Comparative Example 1]

As a comparative example, mesenchymal cells were seeded into a 96-well plate at 5.0 × 10³ cells./well followed by the addition of DMEM (containing 10% FBS and 1% P/S) ad monolayer culturing for 3 days.

### [Comparative Example 2]

### (1) Spheroid Preparation

As a comparative example, spheroids of mesenchymal cells were prepared at 5.0 × 103 cells/spheroid followed by the addition of DMEM (containing 10% FBS and 1% P/S) and culturing for 3 days.

### (2) Observation Results

The results of observing the spheroids three days after the start of culturing using a phase-contrast microscope (IX-71, Olympus) are shown in FIG 2(A).

Based on FIG 2(A), the spheroids three days after the start of culturing were confirmed to have a diameter of about 200 µm.

### (3) HE Staining of Spheroids

### (3-1) Preparation of Spheroid Sections

Sections of the spheroids were prepared using the same method as that of section (5-1) of Example 1.

### (3-2) HE staining

The spheroids were stained with HE stain using the same method as section (5-2) of Example 1. The results of observing the spheroids after staining with a phase-contrast microscope (IX-71, Olympus) are shown in FIG 2(B).

Based on FIG 2(B), cells were confirmed to be densely aggregated in the spheroids.

### [Test Example 1]

### Evaluation Test of Alkaline Phosphatase (ALP) Activity

### (1) Alkaline Phosphatase (ALP) Staining

The cell-embedded beads for hair regeneration of Example 1, the monolayer culture of mesenchymal cells of Comparative Example 1, and the spheroids of mesenchymal cells of Comparative Example 2 were immersed in citric acid-acetone fixative for 30 seconds and then rinsed with deionized water for 45 seconds. Next, the specimens were stained for 30 minutes under normal temperature conditions and under protection from light using a staining solution (consisting of a mixture of 1 capsule of Fast Blue RR Salt Capsule (Sigma), 48 mL of deionized water and 2 mL of Naphthol AS-MX Phosphate Alkaline Solution (Sigma). Next, the specimens were rinsed with deionized water for 120 seconds. Next, the ALP-stained cell-embedded beads for hair generation of Example 1, the monolayer culture of mesenchymal cells of Comparative Example 1 and the spheroids of mesenchymal cells of Comparative Example 2 were respectively observed using a phase-contrast microscope (IX-71, Olympus). The results are shown in FIG. 3.

Based on FIG 3, hardly any cells in which ALP, which is known as a marker protein of hair papilla cells, was activated were observed in the monolayer culture. On the other hand, ALP activity was observed in the spheroids and cell-embedded beads for hair regeneration. In particular, the cell-embedded beads for hair regeneration were observed to exhibit potent ALP activity.

### (2) Quantification of ALP Using Bessey-Lowry Method

### (2-1) Cell Disruption and Preparation of Cell Extract

The cell-embedded beads for hair regeneration of Example 1, the monolayer culture of mesenchymal cells of Comparative Example 1 and the spheroids of mesenchymal cells of Comparative Example 2 were respectively washed twice with PBS(-) followed by the addition of 1 mL of buffer (50 mmol/L Tris/HCl, pH 7.6, 0.1% (v/v) Triton X-100) and placing in frozen storage at -80°C. Next, after repeating freezing and thawing three times, the specimens were homogenized using a disposable homogenizer (Asone). Next, the cell membrane was disrupted by subjecting to ultrasonic treatment for 30 seconds and repeatedly vortexing twice for 10 seconds. Next, the specimens were centrifuged for 10 minutes at 1300 rpm to obtain cell extracts.

### (2-2) Preparation of Dilution Standard Solutions

A standard solution (0.5 mmol/L p-nitrophenol solution) was serially diluted two-fold to prepare a series of serial two-fold dilutions of 0.5 mmol/L, 0.25 mmol/L, 0.125 mmol/L and 0.625 mmol/L.

### (2-3) ALP Reaction

20 µl each of the cell extracts obtained in section (2-1) were removed and added to a 96-well plate. 20 µL aliquots of the dilution standard solutions prepared in section (2-2) were added to the well at the end of each row as the standard row. Next, 100 µL aliquots of substrate buffer solution (consisting of a mixture of 6.7 mmol/L disodium p-nitrophenol phosphate and 2.0 mmol/L magnesium chloride) were added to all wells. Next, after stirring for 1 minute with a microplate stirrer while protecting the well plates from light, the plates were incubated for 15 minutes at 37°C. Subsequently, 80 µL aliquots of a reaction stopping solution (0.2 mmol/L sodium hydroxide) to each well to terminate the reaction.

### (2-4) Analysis

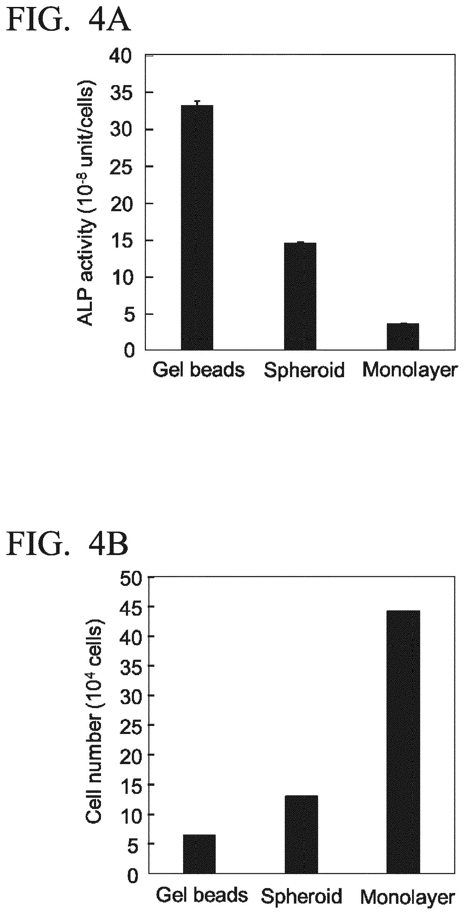

Next, after stirring for 1 minute with a microplate reader (Tecan), absorption of each cell extract at 405 nm was measured using the microplate reader followed by calculation of the ALP activity in each cell extract. The results are shown in FIG. 4A. In FIG 4A, "Gel beads" indicates the cell-embedded beads for hair regeneration of Example 1, "Spheroid" indicates the spheroids of mesenchymal cells of Comparative Example 2, and "Monolayer" indicates the monolayer culture of mesenchymal cells of Comparative Example 1.

In addition, FIG 4B is graph indicating the results of measuring the cell number of the cell-embedded beads for hair regeneration, spheroids and monolayer culture after suspension-culturing for 3 days prior to preparation of the cell extracts.

### (2-5) Results and Discussion

Based on FIG 4A, the potency of ALP activity was determined to be in the order of "Gel beads" (cell-embedded beads for hair regeneration of Example 1) > "Spheroid" (spheroids of mesenchymal cells of Comparative Example 2) > "Monolayer" (monolayer culture of mesenchymal cells of Comparative Example 1).

In addition, based on FIG 4B, the cell number after culturing for 3 days during which the seeded cell numbers were equal was determined to be in the order of "Gel beads" (cell-embedded beads for hair regeneration of Example 1) < "Spheroid" (spheroids of mesenchymal cells of Comparative Example 2) < "Monolayer" (monolayer culture of mesenchymal cells of Comparative Example 1).

The cell doubling time (time required for the cell number to double) of mesenchymal cells has been reported to be 24 hours. In the case of the monolayer culture of mesenchymal cells of Comparative Example 1, the cells grew at a cell doubling time of about 24 hours as reported (increasing from 5.0 × 10³ cells at the start of culturing to 4.5 × 10⁴ cells after culturing for 3 days).

On the other hand, there were hardly increases observed in cell number for the cell-embedded beads for hair regeneration of Example 1 and the spheroids of mesenchymal cells of Comparative Example 2.

In general, mesenchymal cells are known to lose ALP activity, which is a marker for hair papilla cells, each time cell growth progresses (each time the number of passages increases).

Based on these results, since ALP activity increased in the order of the cell-embedded beads for hair regeneration of Example 1, the spheroids of mesenchymal cells of Comparative Example 2 and the monolayer culture of mesenchymal cells of Comparative Example 1, it was surmised that ALP activity was maintained as a result of the cell-embedded beads for hair regeneration of Example 1 having suppressed cell growth.

### [Test Example 2] PCR Analysis of Versican Gene

An RT-PCR analysis of Versican gene, which is a marker protein of hair growth, was carried out using the cell-embedded beads for hair regeneration of Example 1 and the spheroids of mesenchymal cells of Comparative Example 2.

### (1) RNA Extraction

First, the cell-embedded beads for hair regeneration of Example 1 and the spheroids of mesenchymal cells of Comparative Example 2 were respectively collected in 15 mL tubes and once the cell-embedded beads for hair regeneration of spheroids had settled, the supernatant medium was removed to as to leave 1 mL of solution. Next, the 1 mL of solution containing the cell-embedded beads for hair generation or the spheroids was transferred to a 1.5 mL microtube.

Next, the microtubes were centrifuged for 3 minutes at 4°C and 5000 rpm. This procedure was carried out simultaneous to precipitating the cell-embedded beads for hair generation or spheroids to precool the inside of the centrifuge tubes in order to discard the remaining medium. Next, after discarding the supernatant (medium), 350 µL of Buffer RLT were added and adequately pipetted. Next, the pipetted solutions were collected in a QIA Shredder spin column and centrifuged for 2 minutes at 4°C and 10000 rpm. Next, the upper portion of the QIA Shredder spin column was discarded followed by adding 350 µL of 70% ethanol to the solutions inside the collection tubes and transferring to an RNeasy spin column. Next, the collection tubes were centrifuged for 15 seconds at 4°C and 10000 rpm. Next, the filtrate inside the collection tubes was discarded followed by adding 700 µL of Buffer RW1 and centrifuging for 15 seconds at 4°C and 10000 rpm. Next, the filtrate in the collection tubes was discarded followed by adding 500 µL of Buffer RPE and centrifuging for 15 seconds at 4°C and 10000 rpm. Next, the filtrate in the collection tubes was discarded followed by adding 500 µL of Buffer RPE and centrifuging for 2 minutes at 4°C and 10000 rpm. Next, the columns were transferred to new 2 mL collection tubes following centrifugation and centrifuged for 1 minute at 4°C and 10000 rpm. This was done to remove the remaining Buffer RPE. Next, after transferring the columns to 1 mL microtubes following centrifugation, 30 µL of RNase-free water were added and centrifuged for 1 minute at 4°C and 10000 rpm. Next, 30 µL of RNase-free water were again added to 1 mL microtubes in which the centrifuged columns were installed followed by centrifuging for 1 minute at 4°C and 10000 rpm to obtain RNA lysates.

### (2) Measurement of RNA Concentration Using Spectrophotometer

Next, a Nano Vue power supply was attached and the dilatation factor was set to 60.0. Here, dilution factor refers to the final volume during RNA extraction. Next, after wiping the measuring plate with 70% ethanol, 20 µL of RNase-free water were applied to the center of the measuring plate following by pressing the "OA/100%T" button. This procedure was used to obtain a baseline. Next, 2 µL of the RNA lysates obtained in section (1) were applied to the center of the measuring plate followed by pressing the "Measure" button. A260/280 represents the purity of the sample and is preferably near 2.0. Next, the measuring plate was wiped with 70% ethanol with a Kimwipe prior to use when measuring the next sample (RNA lysate).

The concentration of the RNA lysate for RNA derived from the cell-embedded beads for hair regeneration was 311 µg/mL, while the concentration of RNA lysate for RNA derived from the spheroids was 344 µg/mL.

### (3) RT-PCR

Next, the RNA lysates for which concentration was measured in section (2) were diluted to 150 µg/mL and incubated for 5 minutes at 65°C followed by cooling on ice. Next, a solution having the composition shown in the following Table 1 was added to the microtubes followed by covering the microtubes with a clear film. In Table 1, RNA refers to the RNA lysate derived from the cell-embedded beads for hair regeneration or the RNA lysate derived from the spheroids.

**[Table 1]**

| Type | Amount (µL) |
|---|---|
| Nuclear-free water | 12 |
| 5XRT buffer | 4 |
| Primer mix | 1 |
| Enzyme mix | 1 |
| RNA | 2 |
| Total | 20 |

Next, the microtubes were placed in a thermal cycler and the thermal cycler was confirmed to be securely closed. Next, a reverse transcription reaction was carried out for 5 minutes at 98°C to respectively obtain cDNA that is the reverse transcription product of the RNA derived from the cell-embedded beads for hair regeneration and cDNA that is the reverse transcription product of RNA derived from the spheroids.

Next, a solution having the composition shown in the following Table 2 was added to the microtubes and the microtubes were covered with a clear film. In Table 2, DNA indicates cDNA that is the reverse transcription product of the RNA derived from the cell-embedded beads for hair regeneration or cDNA that is the reverse transcription product of the spheroids. Moreover, the base sequences of the primers used in PCR are shown in Table 3.

**[Table 2]**

| Type | Amount (µL) |
|---|---|
| SYBR Green Master Mix | 10 |
| Forward primer | 0.4 |
| Reverse primer | 0.4 |
| Dye | 0.4 |
| Nuclear-free water | 7.8 |
| DNA | 1 |
| Total | 20 |

**[Table 3]**

| | Forward primer | SEQ ID NO. | Reverse primer | SEQ ID NO. |
|---|---|---|---|---|
| Versican | 5'-GACGACTGTCTTGGTGG-3 | 1 | 5'-ATATCCAAACAAGCCTG-3' | 2 |
| GAPDH | 5'-AGAACATCATCCCTGCATCC-3' | 3 | 5'-TCCACCACCCTGTTGCTGTA-3' | 4 |

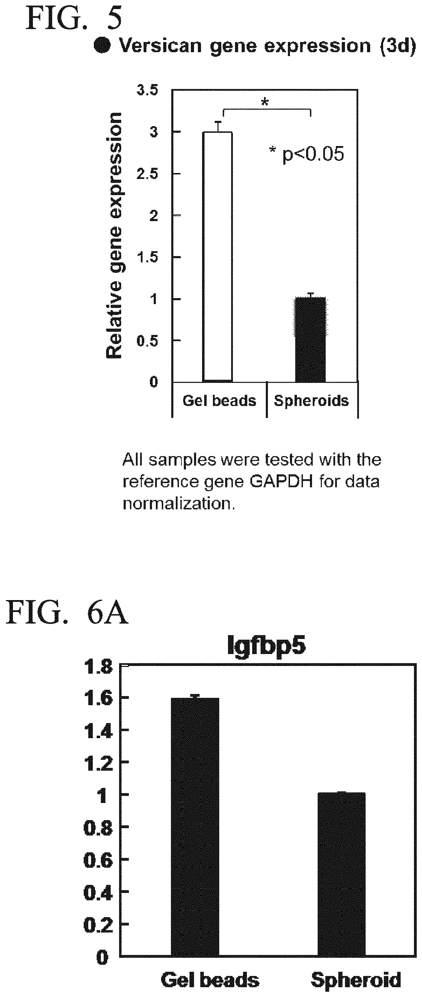

Next, the microtubes were placed in a thermal cycler and the thermal cycler was confirmed to be securely closed. Next, PCR was carried out using a protocol consisting of 4 minutes at 95°C (5 seconds at 95°C, 60 seconds at 60°C) × 45 cycles and 10 minutes at 72°C. Expression of GAPDH was measured as a control and the relative expression of Versican relative to the expression of GAPDH was calculated. The results are shown in FIG 5. In FIG 5, "Gel beads" indicates the cell-embedded beads for hair regeneration of Example 1 while "Spheroid" indicates the spheroids of mesenchymal cells of Comparative Example 2.

Based on FIG 5, with respect to expression of Versican gene, the cell-embedded beads for hair regeneration of Example 1a was clearly determined to by improved roughly three-fold in comparison with the spheroids of mesenchymal cells of Comparative Example 2.

In general, expression of Versican by mesenchymal cells is known to decrease each time growth progresses (each time the number of passages increases). In addition, expression of Versican gene has been reported to be able to be maintained by spheroid culturing in comparison with monolayer culturing on a tissue dish.

According to these results, the cell-embedded beads for hair regeneration of Example 1 demonstrated expression of Versican gene that was higher than that of the spheroids of mesenchymal cells of Comparative Example 2, and culturing of mesenchymal cells using the cell-embedded beads for hair regeneration of Example 1 was suggested have the potential for being a more suitable mesenchymal cell culture method than existing methods for culturing mesenchymal cells using spheroids.

### [Test Example 3] PCR Analysis of Igfbp5 Gene and Tgfb2 Gene

Expression of marker genes related to hair growth was evaluated using the cell-embedded beads for hair regeneration of Example 1 and the spheroids of mesenchymal cells of Comparative Example 2.

### (1) RNA Extraction

RNA lysates were obtained from the cell-embedded beads for hair regeneration of Example 1 and the spheroids of mesenchymal cells of Comparative Example 2 using the same method as in section (1) of Test Example 2.

### (2) Measurement of RNA Concentration Using Spectrophotometer

Next, the concentrations of the RNA lysates of the cell-embedded beads for hair regeneration of Example 1 and the spheroids of mesenchymal cells of Comparative Example 2 were measured using the same method as in section (2) of Text Example 2.

The concentration of the RNA lysate derived from the cell-embedded beads for hair regeneration was 398 µg/mL while the concentration of the RNA lysate derived from the spheroids was 279 µg/mL.

### (3) RT-PCR

Reverse transcription reactions were carried out using the same method as in section (3) of Test Example 2 and a reverse transcription product in the form of cDNA derived from the cell-embedded beads for hair generation of a reverse transcription product in the form of cDNA derived from the spheroids was obtained. Next, PCR reactions were carried out using the same method as in section (3) of Test Example 2 with the exception of using the forward primers and reverse primers for amplifying Igfgp5 gene and Tgfb2 gene indicated in the following Table 4 instead of the forward primers and reverse primers for amplification of Versican, and the relative expression of Igfgp5 or Tgfb2 relative to the expression of GAPDH was calculated. The results are shown in Tables 6A and 6B.

**[Table 4]**

| | Forward primer | SEQ ID NO: | Reverse primer | SEQ ID NO: |
|---|---|---|---|---|
| Igfbp5 | 5'-ATGAGACAGGAATCCGAACA-3' | 5 | 5'-TCAACGTTACTGCTGTCGAA-3' | 6 |
| Tgfb2 | 5'-TCCCGAATAAAAGCGAAGAG-3' | 7 | 5'-AAGCTTCGGGATTTATGGTG-3' | 8 |

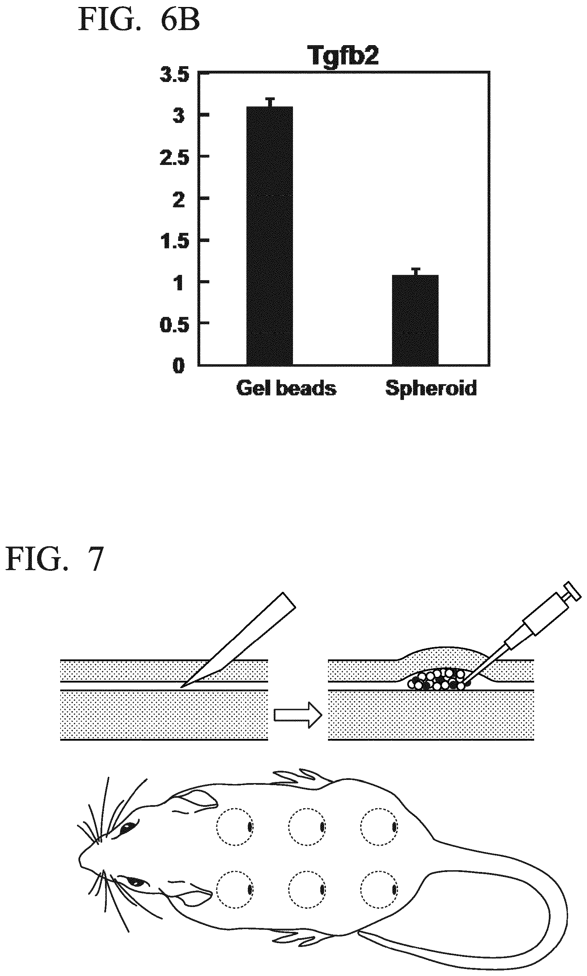

Based on FIGS. 6A and 6B, expression of Igfbp5 gene and expression of Tgfb2 gene by the cell-embedded beads for hair regeneration of Example 1 were clearly determined to improve by about 1.6-fold to 3-fold in comparison with the spheroids of Comparative Example 2.

Expression of these genes has been reported in the prior art to increase significantly as a result of spheroid culturing of mouse mesenchymal cells in comparison with monolayer culturing.

According to these results, culturing of mesenchymal cells using the cell-embedded beads for hair regeneration of Example 1 demonstrated higher expression of Igfbp5 gene and Tgfb2 gene than existing methods for culturing mesenchymal cells using spheroids.

In addition, based on Test Example 2, the cell-embedded beads for hair regeneration of Example 1 were clearly determined to demonstrate higher expression of Versican gene than the spheroids of mesenchymal cells of Comparative Example 2.

On the basis of these results, the method of culturing mesenchymal cells using the cell-embedded beads for hair regeneration of Example 1 was suggested to be a culture method for expressing a higher level of hair regeneration activity in comparison with existing methods for culturing mesenchymal cells using spheroids.

Examples of possible factors behind this include the presence of a collagen-rich environment, cell-embedded beads having an improved oxygen supply in comparison with spheroids, and the contribution of mechanical stimulation to cells in the aggregation step during bead production.

### [Test Example 4] Subcutaneous Transplantation Test Using Nude Mouse

### (1) Subcutaneous Transplantation to Nude Mouse

The cell-embedded beads for hair regeneration of Example 1 and the spheroids of mesenchymal cells of Comparative Example 2 were transplanted into the skin of a nude mouse using the patch method. Animal management and animal experiments were carried out while strictly observing the guidelines of the Animal Care and Use Committee of Yokohama National University.

More specifically, the nude mouse was first anesthetized by inhaling isoflurane followed by disinfecting the back of the animal with Isodine. Next, an incision for transplantation extending from the skin epidermal layer to the subdermal layer was formed using a V-lance microscalpel (Alcon Japan) (see left side of FIG 7). Next, 30 beads/location of the cell-embedded beads for hair regeneration of Example 1 or 30 spheroids/location of the spheroids of mesenchymal cells of Comparative Example 2 were mixed with 1.0 × 10⁶ cells/site of epithelial cells harvested from a mouse fetus within the uterus of a pregnant mouse (C57BL/6jjcl) and inserted into the incision for transplantation at a total of three locations (see right side of FIG 7).

Furthermore, a mixture of the cell-embedded beads for hair regeneration of Example 1 and epithelial cells was transplanted at three locations on the left side of a single nude mouse, and a mixture of the spheroids of mesenchymal cells of Comparative Example 2 were transplanted at three locations on the right side. The state of regenerated hair at the transplantation portions of the nude mouse one month after transplanting the mixture of the cell-embedded beads for hair regeneration of Example 1 and the epithelial cells is shown in FIG 8. In addition, the state of regenerated hair at the transplantation portions of the nude mouse one month after transplanting the mixture of the spheroids of mesenchymal cells of Comparative Example 2 and the epithelial cells is shown in FIG 9. Furthermore, (A) and (B) in FIGS. 8 and 9 are images of regenerated hair at transplantation portions photographed using a digital microscope (VHX-1000, Keyence). (C) indicates an image of the state after having extracted hair within (A) as photographed using a digital microscope (VHX-1000, Keyence). (D) to (F) indicate images of regenerated hair photographed using a scanning electron microscope (SEM).

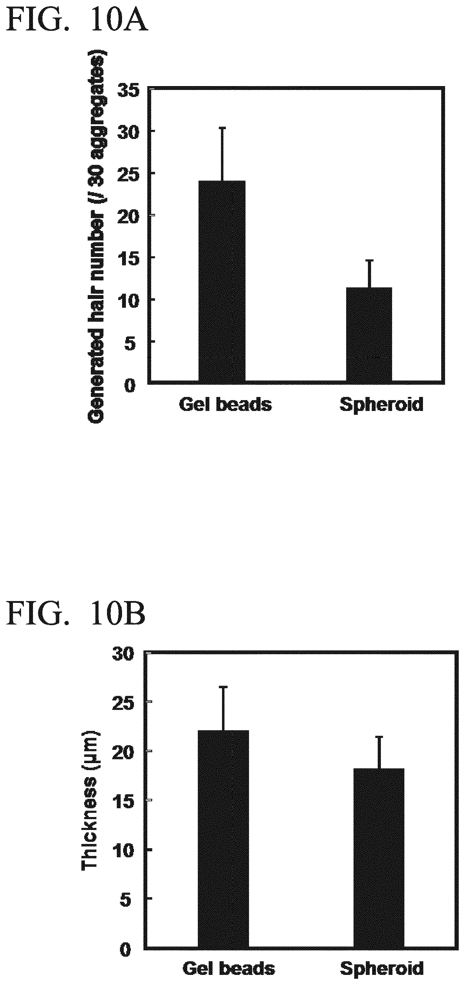

In addition, FIG 10 shows graphs indicating the regenerated hair number (see FIG 10A) and the thickness of the regenerated hair (see FIG 10B) at the transplantation portions of a nude mouse one month after transplanting a mixture of the cell-embedded beads for hair regeneration and epithelial cells or a mixture of the spheroids of mesenchymal cells of Comparative Example 2 and epithelial cells.

Based on FIGS. 8 and 9, hairs were formed as black masses at the transplantation portions of the mixture of the cell-embedded beads for hair regeneration of Example 1 and the epithelial cells and a large amount of hairs were observed within the black masses.

On the other hand, there were few regenerated hair at the transplantation portions of the mixture of the spheroids of mesenchymal cells of Comparative Example 2 and the epithelial cells, the size of the black masses was observed to be smaller.

In addition, morphologically normal hairs having a cuticle structure were formed for each of these hairs.

As shown in FIG 10, analysis of the respect regenerated hair numbers indicated that the regenerated hair number at transplantation portions of the mixture of the cell-embedded beads for hair regeneration of Example 1 and the epithelial cells was nearly two times more in comparison with the transplantation portions of the mixture of the spheroids of mesenchymal cells of Comparative Example 2 and the epithelial cells.

In addition, the thickness of the regenerated hairs was 20 µm in all cases, and was roughly equal to that of mouse body hair.

On the basis of the above results, the cell-embedded beads for hair regeneration of the present teaching were indicated to be able to realize higher hair regeneration efficiency than spheroids.

### [Example 2] Preparation of Cell-Embedded Beads for Mouse Hair Regeneration 2

### (1) Collection of Mesenchymal Cells and Epithelial Cells

Mesenchymal cells were collected using the same method as section (1) of Example 1 from an 18-day-old mouse fetus within the uterus of a pregnant C57BL/6jjcl mouse.

### (2) Cell-Embedded Cured Gel Formed Body Preparation Step

Next, Dulbecco's Modified Eagle Medium (DMEM, containing 10% FBS and 1% (P/S)) was added to 1.0 × 10⁶, 2.0 × 10⁶, 4.0 × 10⁶ or 8.0 × 10⁶ of the collected mesenchymal cells followed by removing the supernatant after centrifuging for 180 seconds at 1000 rpm. Next, the mesenchymal cells were suspended in 0.2 mL of collagen type 1 (containing 3 mg/mL of collagen, Nitta Gelatin).

The suspension was dropped 2 µL at a time (0.5 × 10⁴ cells/2 µL, 1.0 × 10⁴ cells/2 µL, 2.0 × 10⁴ cells/2 µL or 4.0 × 10³ cells/2 µL) onto the back of a culture dish cover (water-repellent surface). The suspension was then incubated for 30 minutes at 37°C to prepare the cell-embedded cured gel formed body.

### (3) Aggregation Step

Next, the cell-embedded cured gel formed product was detached from the back of the culture dish cover using Pipetman. Next, the cell-embedded cured gel formed body was placed in a culture dish followed by the addition of DMEM (containing 10% FBS and 1% P/S) and suspension-culturing for 3 days to prepare cell-embedded beads for hair regeneration.

### (4) Observation Results

The results of observing the cell-embedded beads for hair regeneration three days after the start of culturing using a phase-contrast microscope (IX-71, Olympus) are shown in FIG 11A.

Based on FIG 11A, a tendency was observed for bead diameter to become smaller as the number of embedded cells increased.

### (5) Measurement of Diameter of Cell-Embedded Beads for Hair Regeneration

Images of the cell-embedded beads for hair regeneration on day 0, day 1 and day 3 from the start of culturing were acquired using a phase-contrast microscope. Next, the images were analyzed using Image J followed by measurement of the diameter of the cell-embedded beads for hair regeneration. The results are shown in FIG 11B.

Based on FIG 11B, as a result of culturing for 3 days, the diameter of the cell-embedded beads for hair regeneration changed dynamically and the diameter thereof was 500 µm to 800 µm.

In addition, a tendency was observed for bead diameter to become smaller as the number of embedded cells increased. This is thought to have been caused by aggregation of collagen fibers within the beads attributable to the tractive force of the cells, and the force responsible for aggregating the collage fibers is thought to have increased due to the increase in cell number.

### [Test Example 5] PCR Analysis of Versican Gene and HIF1α Gene

Versican gene, which is a marker protein for hair growth, and HIF1α gene, which is a marker for hypoxia, were analyzed by PCR using cell-embedded beads for hair regeneration having different numbers of the mesenchymal cells of Example 2.

### (1) RNA Extraction

First, RNA lysates were obtained for the cell-embedded beads for hair regeneration having different numbers of the mesenchymal cells of Example 2 using the same method as section (1) of Test Example 2.

### (2) Measurement of RNA Concentration Using Spectrophotometer

Next, the concentrations of the RNA lysates of each of the cell-embedded beads for hair regeneration were measured using the same method as section (2) of Test Example 2.

### (3) RT-PCR

Next, RNA reverse transcription products in the form of cDNA derived from each of the cell-embedded beads for hair regeneration were obtained using the same method as section (3) of Test Example 2.

Next, a solution having the composition shown in the aforementioned Table 2 was added to the microtubes followed by covering the microtubes with a clear film. In Table 2, DNA refers to the RNA reverse transcription product in the form of cDNA derived from each of the cell-embedded beads for hair regeneration. Moreover, the base sequences of the primers used in PCR are shown in Table 5.

**[Table 5]**

| | Forward primer | SEQ ID NO: | Reverse primer | SEQ ID NO: |
|---|---|---|---|---|
| Versican | 5'-GACGACTGTCTTGGTGG-3' | 1 | 5'-ATATCCAAACAAGCCTG-3' | 2 |
| HIF1α | 5'-GGAGATCCTTCGAGGAGCACTT-3' | 9 | 5'-GGCGATTTAGCAGCAGATATAAGAA-3' | 10 |
| GAPDH | 5'-AGAACATCATCCCTGCATCC-3' | 3 | 5'-TCCACCACCCTGTTGCTGTA-3' | 4 |

Next, the microtubes were placed in a thermal cycler and the thermal cycler was confirmed to be securely closed. PCR was carried out using a protocol consisting of 4 minutes at 95°C (5 seconds at 95°C, 60 seconds at 60°C) × 45 cycles and 10 minutes at 72°C. Expression of GAPDH was measured as a control and the relative expression of Versican or HIF1α relative to the expression of GAPDH was calculated. The results are shown in FIG 12(i) for Versican and FIG 12(ii) for HIF1α.

Based on FIG 12(i), the cell-embedded beads for hair generation, in which the cell number of mesenchymal cells was 1 × 10⁴ cells/bead, demonstrated the highest expression of Versican gene. In addition, although the expression of the hypoxia marker, HIF1α gene, decreased accompanying an increase in the cell number of mesenchymal cells within the beads, the expression level of Versican gene decreased.

On the basis of the above, a decrease in hair growth activity attributable to hypoxia was suggested in a portion of the cells as a result of making the cell number of mesenchymal cells in the cell-embedded beads for hair regeneration to be greater than 1 × 10⁴ cells/bead (cell density: approximately 1 × 10⁵ cells/cm³). Therefore, a cell number of 1 × 10⁴ cells/bead was judged to be the number of embedded cells that demonstrates the highest ability to induce hair follicles without causing the mesenchymal cells in the cell-embedded beads for hair regeneration to become hypoxic, and subsequent experiments were conducted on the basis of this finding.

### [Example 3] Preparation of Cell-Embedded Beads for Mouse Hair Regeneration 3

### (1) Collection of Mesenchymal Cells

Mesenchymal cells were collected using the same method as section (1) of Example 1 from an 18-day-old mouse fetus within the uterus of a pregnant C57BL/6jjcl mouse.

### (2) Preparation of Collagen Solutions

3 mg/mL of collagen type 1 (Nitta Gelatin) were diluted with hydrochloric acid having a pH of 3 as necessary to prepare a collagen solution having a concentration of 0.75 mg/mL, 1.50 mg/mL, 2.25 mg/mL or 3.00 mg/mL.

### (3) Cell-Embedded Cured Gel Formed Body Preparation Step

Next, Dulbecco's Modified Eagle Medium (DMEM, containing 10% FBS and 1% (P/S)) was added to 1.0 × 10⁶ of the collected mesenchymal cells followed by removing the supernatant after centrifuging for 180 seconds at 1000 rpm. Next, the mesenchymal cells were suspended in the 2 mL of the collagen solution having a concentration of 0.75 mg/mL, 1.50 mg/mL, 2.25 mg/mL or 3.00 mg/mL 0.2 mL of collagen type 1 prepared in section (2).

100 drops of the suspension were dropped 2 µL at a time (1.0 × 10⁴ cells/2 µL,) onto the back of a culture dish cover (water-repellent surface). The suspension was then incubated for 30 minutes at 37°C to prepare the cell-embedded cured gel formed body.

### (4) Aggregation Step

Next, the cell-embedded cured gel formed product was detached from the back of the culture dish cover using Pipetman. Next, the cell-embedded cured gel formed body was placed in a culture dish followed by the addition of DMEM (containing 10% FBS and 1% P/S) and suspension-culturing for 3 days to prepare cell-embedded beads for hair regeneration.

### (5) Observation Results

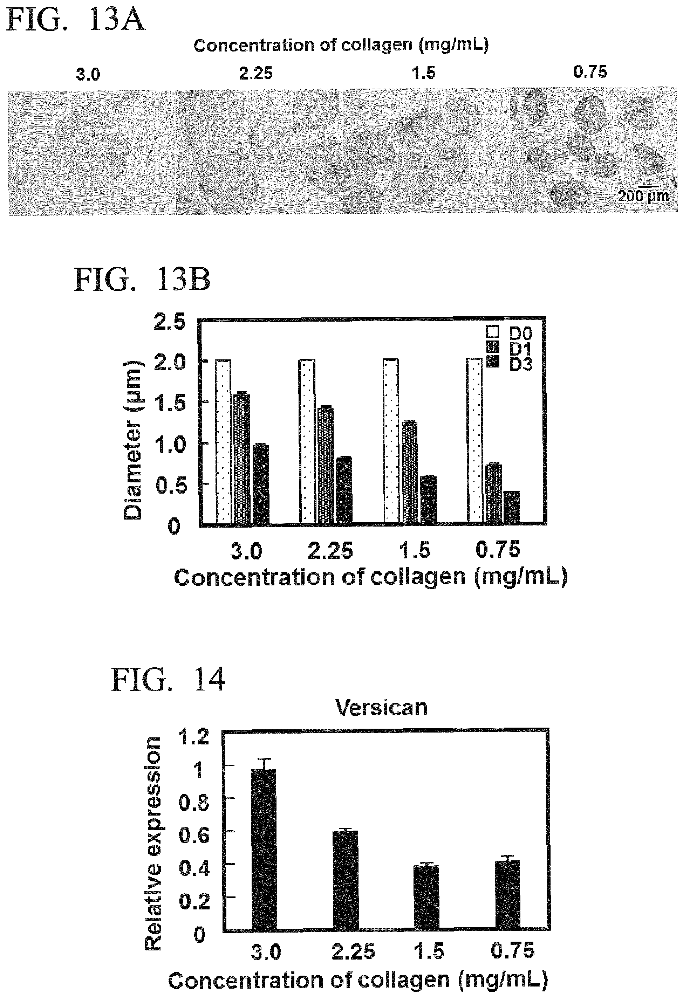

The results of observing the cell-embedded beads for hair regeneration three days after the start of culturing using a phase-contrast microscope (IX-71, Olympus) are shown in FIG 13A.

Based on FIG 11A, a tendency was observed for bead diameter to become larger as collagen concentration increased.

### (6) Measurement of Diameter of Cell-Embedded Beads for Hair Regeneration

Images of the cell-embedded beads for hair regeneration on day 1 and day 3 from the start of culturing were acquired using a phase-contrast microscope. Next, the images were analyzed using Image J followed by measurement of the diameter of the cell-embedded beads for hair regeneration. The results are shown in FIG 13B.

Based on FIG 13B, as a result of culturing for 3 days, the diameter of the cell-embedded beads for hair regeneration changed dynamically and the diameter thereof was 300 µm to 900 µm.

In addition, a tendency was observed for bead diameter to become larger as collagen concentration increased. This is thought to have been caused by embrittlement of gel crosslinks the lower the collagen concentration, or in other words, the lower the density of collagen fibers, and this is thought to have made it possible for the tractive force of the cells to facilitate contraction of the gel.

### [Test Example 6] PCR Analysis of Versican Gene and HIF1α Gene

Versican gene, which is a marker protein for hair growth, was analyzed by PCR using the cell-embedded beads for hair regeneration having different collagen concentrations of Example 3.

### (1) RNA Extraction

First, RNA lysates were obtained for the cell-embedded beads for hair regeneration having different collagen concentrations of Example 3 using the same method as section (1) of Test Example 2.

### (2) Measurement of RNA Concentration Using Spectrophotometer

Next, the concentrations of the RNA lysates of each of the cell-embedded beads for hair regeneration were measured using the same method as section (2) of Test Example 2.

### (3) RT-PCR

Next, RNA reverse transcription products in the form of cDNA derived from each of the cell-embedded beads for hair regeneration were obtained using the same method as section (3) of Test Example 2.

Next, a solution having the composition shown in the aforementioned Table 2 was added to microtubes followed by covering the microtubes with a clear film. In Table 2, DNA refers to the RNA reverse transcription product in the form of cDNA derived from each of the cell-embedded beads for hair regeneration. Moreover, the base sequences of the primers used in PCR are shown in the aforementioned Table 3.

Next, the microtubes were placed in a thermal cycler and the thermal cycler was confirmed to be securely closed. PCR was carried out using a protocol consisting of 4 minutes at 95°C (5 seconds at 95°C, 60 seconds at 60°C) × 45 cycles and 10 minutes at 72°C. Expression of GAPDH was measured as a control and the relative expression of Versican relative to the expression of GAPDH was calculated. The results are shown in FIG 14.

Based on FIG 14, a tendency was observed for expression of the hair growth (follicle induction) marker, Versican, to decrease accompanying reductions in collagen concentration.

On the basis of the above, a concentration of 3.0 mg/ml of collagen composing the cell-embedded beads for hair regeneration demonstrated the highest hair growth activity, while decreases in hair growth activity were indicated to occur by reducing collagen concentration. Therefore, a concentration of 3.0 mg/mL was judged to be suitable for concentration of collagen composing the cell-embedded beads for hair regeneration, and subsequent experiments were conducted at that concentration.

### [Example 4] Preparation of Cell-Embedded Beads for Mouse Hair Regeneration 4

### (1) Collection of Mesenchymal Cells

Mesenchymal cells were collected using the same method as section (1) of Example 1 from an 18-day-old mouse fetus within the uterus of a pregnant C57BL/6jjcl mouse.

### (2) Cell-Embedded Cured Gel Formed Body Preparation Step

Next, DMEM (containing 10% FBS and 1% (P/S)) was added to 1.0 × 10⁶ of the collected mesenchymal cells followed by removing the supernatant after centrifuging for 180 seconds at 1000 rpm. Next, the mesenchymal cells were suspended in the 2 mL of collagen type I (containing 3 mg/mL of collagen, Nitta Gelatin).

100 drops of the suspension were dropped 2 µL at a time (1.0 × 10⁴ cells/2 µL,) onto the back of a culture dish cover (water-repellent surface). The suspension was then incubated for 30 minutes at 37°C to prepare the cell-embedded cured gel formed body.

### (3) Aggregation Step

Next, the cell-embedded cured gel formed product was detached from the back of the culture dish cover using Pipetman. Next, the cell-embedded cured gel formed body was placed in a culture dish followed by the addition of DMEM (containing 10% FBS and 1% P/S) and suspension-culturing for 3 days to prepare cell-embedded beads for hair regeneration.

### (4) Actin and Nuclear Staining

The cell-embedded beads for hair regeneration three days after the start of culturing were fixed with paraformaldehyde and subjected to actin staining and nuclear staining.

First, PBS was added to the cell-embedded beads for hair regeneration followed by allowing to stand undisturbed for 1 minute and removing the PBS. Next, the same procedure was repeated twice to wash the cell-embedded beads for hair regeneration. Next, Triton X-100 was added followed by allowing to stand undisturbed for 10 minutes (penetration). Next, PBS was added followed by allowing to stand undisturbed for 1 minute and removing the PBS. Next, the same procedure was repeated twice to wash the cell-embedded beads for hair regeneration. Next, Rhodamine Phalloidin (Molecular Probes) was added following by allowing to stand undisturbed for 30 minutes to carry out actin staining. Next, PBS was added following by allowing to stand undisturbed for 1 minute and removing the PBS. Next, the same procedure was repeated twice to wash the cell-embedded beads for hair regeneration. Next, 4',6-diamidino-2-phenylindole (DAPI, Wako Pure Chemical Industries) was added followed by allowing to stand undisturbed for 5 minutes to carry out nuclear staining. Next, PBS was added followed by allowing to stand undisturbed for 5 minutes and removing the PBS. Next, the same procedure was repeated twice to wash the cell-embedded beads for hair regeneration.

### (5) Observation Results

The results of observing the cell-embedded beads for hair regeneration three days after the start of culturing using a fluorescence microscope (DP-71, Olympus) and confocal laser microscope (LSM700, Carl Zeiss) are shown in FIG 15A (upper right: fluorescence micrograph, lower right: confocal laser micrograph).

Based on FIG 15A (upper right: fluorescence micrograph, lower right: confocal laser micrograph), mesenchymal cells in the cell-embedded beads for hair regeneration formed a developed actin skeleton.

### (6) Measurement of Diameter of Cell-Embedded Beads for Hair Regeneration

Images of the cell-embedded beads for hair regeneration on day 1 and day 3 from the start of culturing were acquired using a phase-contrast microscope (IX-71, Olympus). Next, the images were analyzed using Image J followed by measurement of the diameter of the cell-embedded beads for hair regeneration. The results are shown in FIG 15B (-).

Based on FIG 15B (-), as a result of culturing for 3 days, the diameter of the cell-embedded beads for hair regeneration changed dynamically and the diameter thereof was about 800 µm.

### [Comparative Example 3]

### (1) Collection of Mesenchymal Cells

### Collection of Mesenchymal Cells

Mesenchymal cells were collected using the same method as section (1) of Example 1 from an 18-day-old mouse fetus within the uterus of a pregnant C57BL/6jjcl mouse.

### (2) Cell-Embedded Cured Gel Formed Body Preparation Step

The cell-embedded cured gel formed body was prepared using the same method as section (2) of Example 4.

### (3) Aggregation Step

Cell-embedded beads for hair regeneration were prepared using the method as section (3) of Example 4 with the exception of using DMEM (containing 10% FBS and 1% P/S) to which 30 uM blebbistatin had been added.

Furthermore, blebbistatin is an inhibitor that inhibits myosin, which is involved in the tractive force of cells, and is known to inhibit the formation of developed actin skeleton.

### (4) Actin and Nuclear Staining

The cell-embedded beads for hair regeneration three days after the start of culturing were subjected to actin and nuclear staining using the same method as section (4) of Example 4.

### (5) Observation Results

The results of observing the cell-embedded beads for hair regeneration three days after the start of culturing using a fluorescence microscope (DP-71, Olympus) and confocal laser microscope (LSM700, Carl Zeiss) are shown in FIG 15A (upper left: fluorescence micrograph, lower left: confocal laser micrograph).

Based on FIG 15A (upper left: fluorescence micrograph, lower left: confocal laser micrograph), mesenchymal cells in the cell-embedded beads for hair regeneration were not observed to form an actin skeleton.

### (6) Measurement of Diameter of Cell-Embedded Beads for Hair Regeneration

Images of the cell-embedded beads for hair regeneration on day 0, day 1 and day 3 from the start of culturing were acquired using a phase-contrast microscope (IX-71, Olympus). Next, the images were analyzed using Image J followed by measurement of the diameter of the cell-embedded beads for hair regeneration. The results are shown in FIG 15B (+).

Based on FIG 15B (+), the diameter of the cell-embedded beads for hair regeneration changed dynamically as a result of culturing for three days and the diameter thereof was about 2.0 mm.

This is surmised to due to inhibition of cell tractive force of the mesenchymal cells as a result of adding blebbistatin.

### [Test Example 7] PCR Analysis of Versican Gene

An RT-PCR analysis of Versican gene, which is a marker protein of hair growth, was carried out using the cell-embedded beads for hair regeneration of Example 4 and Comparative Example 3.

### (1) RNA Extraction

First, RNA lysates were obtained for the cell-embedded beads for hair regeneration of Example 4 and Comparative Example 3 using the same method as section (1) of Test Example 2.

### (2) Measurement of RNA Concentration Using Spectrophotometer

Next, the concentrations of the RNA lysates of each of the cell-embedded beads for hair regeneration were measured using the same method as section (2) of Test Example 2.

### (3) RT-PCR

Next, RNA reverse transcription products in the form of cDNA derived from each of the cell-embedded beads for hair regeneration were obtained using the same method as section (3) of Test Example 2.

Next, the solution having the composition shown in the aforementioned Table 2 was added to microtubes and the microtubes were covered with a clear film. In Table 2, DNA refers to the RNA reverse transcription product in the form of cDNA derived from each of the cell-embedded beads for hair regeneration. Moreover, the base sequences of the primers used in PCR are shown in the aforementioned Table 3.

Next, the microtubes were placed in a thermal cycler and the thermal cycler was confirmed to be securely closed. Next, PCR was carried out using a protocol consisting of 4 minutes at 95°C (5 seconds at 95°C, 60 seconds at 60°C) × 45 cycles and 10 minutes at 72°C. Expression of GAPDH was measured as a control and the relative expression of Versican relative to the expression of GAPDH was calculated. The results are shown in FIG 16. In FIG 16, (+) indicates the cell-embedded beads for hair regeneration of Comparative Example 3 that were cultured after adding blebbistatin, while (-) indicates the cell-embedded beads for hair regeneration of Example 4 that were cultured in the absence of the addition of blebbistatin.

Based on FIG 16, a greater tendency for expression of Versican to decrease significantly was observed in the case of blebbistatin (+) (Comparative Example 3) than in the case of blebbistatin (-) (Example 4) on both day 1 and day 3 from the start of culturing. This is thought to be the result of inhibition of contraction of the cell-embedded beads for hair regeneration attributable to the addition of blebbistatin having suppressed expression of follicle-inducing gene (Versican).

On the basis of the above, contraction of the cell-embedded beads for hair regeneration was suggested to have the effect of increasing expression of hair follicle-inducing gene by mesenchymal cells.

### [Example 5] Preparation of Mixture of Cell-Embedded Beads for Mouse Hair Regeneration and Epithelial Cells

### (1) Collection of Mesenchymal Cells and Epithelial Cells

Mesenchymal cells and epithelial cells were collected from an 18-day-old mouse fetus within the uterus of a pregnant C57BL/6jjcl mouse using the same method as section (1) of Example 1.

### (2) Cell-Embedded Cured Gel Formed Body Preparation Step

Next, DMEM (containing 10% fetal bovine serum and 1% (P/S)) was added to 1.0 × 10⁶ of the collected mesenchymal cells followed by removing the supernatant after centrifuging for 180 seconds at 1000 rpm. Next, the mesenchymal cells were suspended in 0.2 mL of collagen type 1 (containing 3 mg/mL of collagen, Nitta Gelatin).

The suspension was dropped 2 µL at a time (1.0 × 10⁴ cells/2 µL) into the wells of a Prime Surface 96 u Plate (Sumitomo Bakelite). The suspension was then incubated for 30 minutes at 37°C to prepare the cell-embedded cured gel formed body.

### (3) Preparation of Cell Suspension of Epithelial Cells

Next, the epithelial cells collected in section (1) were suspended in a 1: 1 mixed medium of DMEM (containing 10% FBS and 1% P/S) and HuMedia-KG2 medium (Kurabo) to 1.0 × 10⁵ cells/mL.

### (4) Cell-Embedded Beads for Hair Regeneration and Epithelial Cell Mixture Preparation Step

Next, 100 µL aliquots of the suspension containing epithelial cells were added to wells containing the cell-embedded cured gel formed body prepared in section (2) followed by suspension-culturing for 3 days to prepare a mixture of the cell-embedded beads for hair regeneration and epithelial cells.

### [Reference Example 1] Preparation of Mouse Regenerated Hair Follicle Primordia

### (1) Collection of Mesenchymal Cells and Epithelial Cells

Mesenchymal cells and epithelial cells were collected from an 18-day-old mouse fetus within the uterus of a pregnant C57BL/6jjcl mouse using the same method as section (1) of Example 1.

### (2) Formation of Hair Follicle Primordia

Next, 0.1 mL of a mixed cell suspension of epithelial cells (1 × 10⁵ cells/mL) and mesenchymal cells (1 × 10⁵ cells/mL) (containing 1 × 10⁴ cells/mL each of epithelial cells and mesenchymal cells) were added to the wells of a Prime Surface 96 u Plate (Sumitomo Bakelite) followed by culturing for 3 days to prepare regenerated hair follicle primordia. A 1:1 mixed medium of DMEM (containing 10% FBS and 1% P/S) and HuMedia-KG2 medium (Kurabo) was used for the medium.

### [Text Example 8] Subcutaneous Transplantation to Nude Mouse

### (1) Subcutaneous Transplantation to Nude Mouse

A mixture of cell-embedded beads for hair regeneration and epithelial cells of Example 5 and the regenerated hair follicle primordia of Reference Example 1 were transplanted into the skin of a nude mouse using the patch method. Animal management and animal experiments were carried out while strictly observing the guidelines of the Animal Care and Use Committee of Yokohama National University.

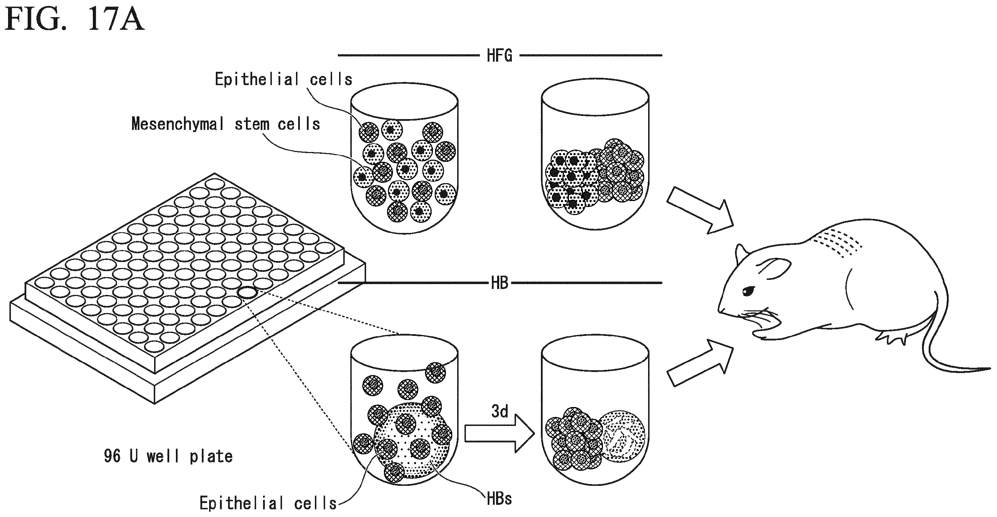

More specifically, the nude mouse was first anesthetized by inhaling isoflurane followed by disinfecting the back of the animal with Isodine. Next, an incision for transplantation extending from the skin epidermal layer to the subdermal layer was formed using a 20 G V-lance microscalpel (Alcon Japan). Next, the mixture of cell-embedded beads for hair regeneration and epithelial cells of Example 5 (1/1 location) (total 50 locations) or each of the regenerated hair follicle primordia of Reference Example 1 (1/1 location) (total 50 locations) was inserted into the incision for transplantation using a micropipette (see FIG 17A).

FIG 17A is a diagram schematically illustrating a method for producing the cell-embedded beads for mouse hair regeneration of Example 5, a method for producing the regenerated hair follicle primordia of Reference Example 1 and the method for subcutaneously transplanting into a nude mouse using the patch method. In FIG 17A, "HB" is the abbreviation for "Hair beads" and indicates the mixture of cell-embedded beads for hair regeneration epithelial cells prepared in Example 5. In addition, "HFG" is the abbreviation for "Hair follicle germs" and indicates the regenerated hair follicle primordia of Reference Example 1.

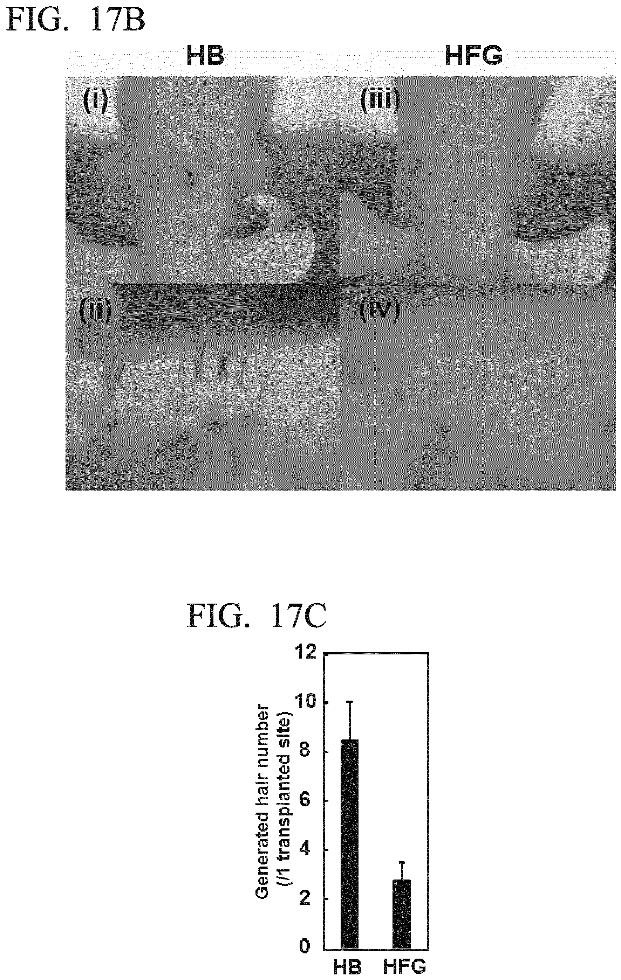

The results of observing the transplantation portions three weeks after transplanting the mixture of cell-embedded beads for hair regeneration and epithelial cells of Example 5 (HB) and regenerated hair follicle primordia (HFG) of Reference Example 1 using a digital microscope (VHX-1000, Keyence) are shown in FIG 17B.

In addition, FIG 17C is a graph indicating the generated hair number at the transplantation portions of the nude mouse three weeks after transplanting the HB of Example 5 and the HFG of Reference Example 1.

### (2) Results

Based on FIG 17B, the mixture of cell-embedded beads for hair regeneration and epithelial cells of Example 5 (HB) and regenerated hair follicle primordia (HFG) of Reference Example 1 each took to the skin of the nude mouse serving as the host and hair growth was observed three weeks after transplant.

In addition, based on FIG 17C, HB was shown to demonstrate a significantly higher generated hair number than HFG

On the basis of the above, HB containing high-density collagen was shown to have a higher ability to generate hair than HFG

### [Example 6] Preparation of Mixture of Cell-Embedded Beads for Human Hair Regeneration and Epithelial Cells

### (1) Collection of Epithelial Cells

Epithelial cells were collected from an 18-day-old mouse fetus within the uterus of a pregnant C57BL/6jjcl mouse using the same method as section (1) of Example 1.

### (2) Cell-Embedded Cured Gel Formed Body Preparation Step

Next, human dermal papilla cell growth medium (DPCGM, PromoCell) was added to 1.0 × 10⁶ human hair papilla cells (PromoCell) followed by removing the supernatant after centrifuging for 180 seconds at 1000 rpm. Next, mesenchymal cells were suspended in 0.2 mL of collagen type 1 (containing 3 mg/mL of collagen, Nitta Gelatin).

The suspension was dropped 2 µL at a time (1.0 × 10⁴ cells/2 µL) into the wells of a Prime Surface 96 u Plate (Sumitomo Bakelite). The suspension was then incubated for 30 minutes at 37°C to prepare the cell-embedded cured gel formed body.

### (3) Preparation of Cell Suspension of Epithelial Cells

Next, the epithelial cells collected in section (1) were suspended in a 1: 1 mixed medium of human dermal papilla cell growth medium (DPCGM, PromoCell) and HuMedia-KG2 medium (Kurabo) to 1.0 × 10⁵ cells/mL.

### (4) Cell-Embedded Beads for Hair Regeneration and Epithelial Cell Mixture Preparation Step

Next, 100 µL aliquots of the suspension containing epithelial cells were added to wells containing the cell-embedded cured gel formed body prepared in section (2) followed by suspension-culturing for 3 days to prepare a mixture of the cell-embedded beads for hair regeneration and epithelial cells.

### [Reference Example 2] Preparation of Regenerated Hair Follicle Primordia Using Human Mesenchymal Cells and Mouse Epithelial Cells

### (1) Collection of Epithelial Cells

Epithelial cells were collected from an 18-day-old mouse fetus within the uterus of a pregnant C57BL/6jjcl mouse using the same method as section (1) of Example 1.

### (2) Formation of Hair Follicle Primordia

Next, 0.1 mL of a mixed cell suspension of epithelial cells (1 × 10⁵ cells/mL) and human hair papilla cells (1 × 10⁵ cells/mL, PromoCell) (containing 1 × 10⁴ cells/mL each of epithelial cells and human hair papilla cells) were added to the wells of a Prime Surface 96 u Plate (Sumitomo Bakelite) followed by culturing for 3 days to prepare regenerated hair follicle primordia. A 1: 1 mixed medium of DMEM (containing 10% FBS and 1% P/S) and HuMedia-KG2 medium (Kurabo) was used for the medium.

### [Test Example 9] Subcutaneous Transplantation Test Using Nude Mouse

### (1) Subcutaneous Transplantation to Nude Mouse

The mixture of cell-embedded beads for hair regeneration and epithelial cells of Example 6 (to be referred to as "HB2") and the regenerated hair follicle primordia of Reference Example 2 (to be referred to as "HFG2") were transplanted into the skin of a nude mouse using the patch method. Animal management and animal experiments were carried out while strictly observing the guidelines of the Animal Care and Use Committee of Yokohama National University.

More specifically, the nude mouse was first anesthetized by inhaling isoflurane followed by disinfecting the back of the animal with Isodine. Next, an incision for transplantation extending from the skin epidermal layer to the subdermal layer was formed using a 20 G V-lance microscalpel (Alcon Japan). Next, the mixture of cell-embedded beads for hair regeneration and epithelial cells of Example 6 (1/1 location) (total 25 locations) or each of the regenerated hair follicle primordia of Reference Example 1 (1/1 location) (total 25 locations) was inserted into the incision for transplantation using a micropipette.

### (2) Observation Results

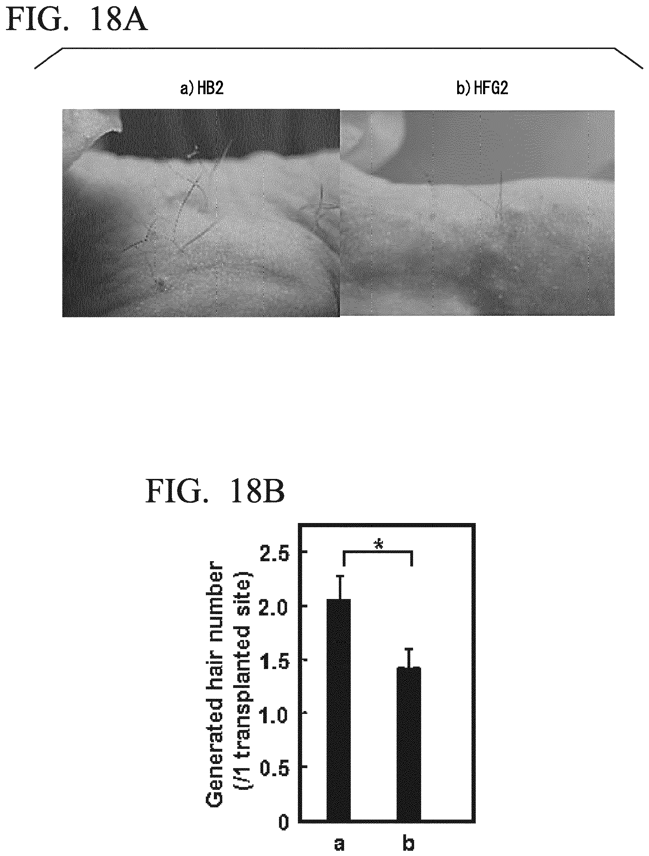

The transplantation portions were observed using a digital microscope (VHX-1000, Keyence) three weeks after transplanting the mixture of cell-embedded beads for hair regeneration and epithelial cells of Example 6 (HB2) and regenerated hair follicle primordia (HFG2) of Reference Example 2. The results are shown in FIG 18A.

In addition, FIG 18B is a graph indicating the generated hair number at the transplantation portions of the nude mouse three weeks after transplanting the HB2 of Example 6 and the HFG2 of Reference Example 2.

### (3) Preparation of Sections

Next, skin of the transplantation portions of the nude mouse was cut out three weeks after transplanting the HB2 of Example 6. Continuing, the tissue was fixed by immersing in 10% neutral buffered formalin solution (Wako) for 1 day. Continuing, the tissue was immersed for 1 hour each in 10%, 20% and 30% sucrose solutions (solutions prepared by diluting Sucrose manufactured by Wako), respectively. The sections replaced with sucrose were gently poured into an embedding agent for preparing frozen tissue sections (Optimal Cutting Temperature Compound (O.C.T. Compound, Sakura Finetek Japan) to enclose the mixed spheroids. Continuing, the sections were cut to an extremely thin thickness using a cryomicrotome. The cut sections were transferred to a slide glass by pressing in a direction perpendicular to the slide glass.

### (4) HE Staining

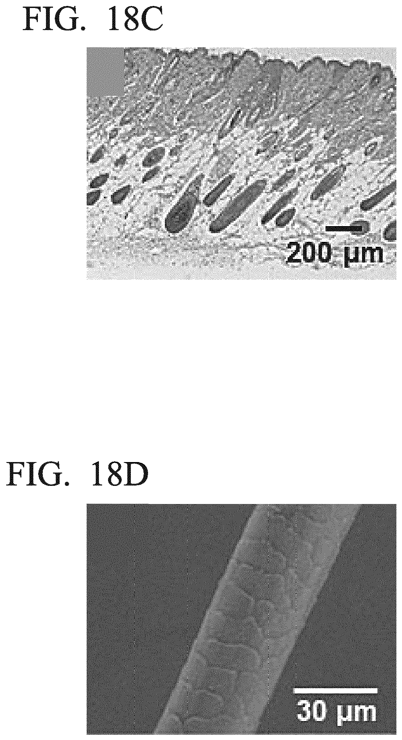

Next, the sections prepared in section (3) were subjected to HE staining using the same method as section (5-2) of Example 1. The results of observing the sections after staining with a phase-contrast microscope (IX-71, Olympus) are shown in FIG 18C.

### (5) Observation of Hair Cuticle Structure

Transplantation portions of the nude mouse three weeks after transplanting the HB2 of Example 6 were observed using a SEM in order to confirm the cuticle structure of the hair. The results are shown in FIG 18D.

### (6) Results and Discussion

Based on FIG 18A, the mixture of cell-embedded beads for hair regeneration and epithelial cells of Example 6 (HB2) and regenerated hair follicle primordia (HFG2) of Reference Example 2 each took to the skin of the nude mouse serving as the host and hair growth was observed three weeks after transplant.

In addition, based on FIG 18B, HB2 exhibited significantly higher values than HFG2 for generated hair number.

In addition, based on FIG 18C, HB2 transplantation portions were able to be confirmed to form hair follicles.

In addition, based on FIG 18D, regeneration of hair having a cuticle structure was able to be confirmed at HB2 transplantation portions.

On the basis of the above, HB2 containing high-density collagen was indicated to have a higher ability to regenerate hair than HFG2.

### Industrial Applicability

According to the present teaching, cell-embedded beads for hair regeneration can be provided that have high hair regeneration activity. Moreover, use of the cell-embedded beads for hair regeneration of the present teaching makes it possible to realize high hair regeneration efficiency.

### Reference Signs List

1: mesenchymal cell, 2: biocompatible hydrogel, 3: support having water-repellent surface, 4: cell-embedded cured gel formed body, 5: medium, 6: cell-embedded beads for hair regeneration

## Claims

1. Cell-embedded beads for hair regeneration comprising:
mesenchymal cells; and
a biocompatible hydrogel at a concentration of 1.0 mg/mL or more and 10 mg/mL or less,
wherein the mesenchymal cells are hair papilla cells, dermal root sheath cells, skin mesenchymal cells in a developmental period or hair follicle mesenchymal cells induced from pluripotent cells.

2. The cell-embedded beads for hair regeneration according to claim 1, comprising:
the mesenchymal cells being hair papilla cells, dermal root sheath cells, skin mesenchymal cells in a developmental period or hair follicle mesenchymal cells induced from pluripotent cells; and
a biocompatible hydrogel of 1.0 mg/mL or more and 3.0 mg/mL or less.

3. The cell-embedded beads for hair regeneration according to claim 1 or 2,
wherein the biocompatible hydrogel is an extracellular matrix component which gelates.

4. The cell-embedded beads for hair regeneration according to claim 3,
wherein the extracellular matrix component is type I collagen.

5. The cell-embedded beads for hair regeneration according to any of claims 1 to 4,
wherein the cell density of the mesenchymal cells is 5 × 10⁴ cells/cm³ or more and 2 × 10⁵ cells/cm³ or less.

6. A method for producing cell-embedded beads for hair regeneration, comprising:
preparing liquid droplets containing mesenchymal cells and a biocompatible hydrogel and curing the biocompatible hydrogel; and
aggregating the cell-embedded cured hydrogel for suspension culturing using the tractive force of the cells to obtain the cell-embedded beads,
wherein the mesenchymal cells are hair papilla cells, dermal root sheath cells, skin mesenchymal cells in a developmental period or hair follicle mesenchymal cells induced from pluripotent cells, and
wherein a concentration of the biocompatible hydrogel in the cell-embedded beads is 1.0 mg/mL or more and 10 mg/mL or less.

7. The method for producing cell-embedded beads for hair regeneration according to claim 6,
wherein, the liquid droplets are prepared by dropping onto a support having a water-repellent surface.

8. The method for producing cell-embedded beads for hair regeneration according to claim 6 or 7,
wherein, the concentration of the mesenchymal cells contained in the liquid droplets is 5 × 10⁵ cells/mL or more and 1 × 10⁸ cells/mL or less.

9. A kit for hair regeneration, comprising:
cell-embedded beads for hair regeneration; and
epithelial cells,
wherein the cell-embedded beads comprising:
mesenchymal cells being hair papilla cells, dermal root sheath cells, skin mesenchymal cells in a developmental period or hair follicle mesenchymal cells induced from pluripotent cells; and
a biocompatible hydrogel of 1.0 mg/mL or more and 10 mg/mL or less.

10. The kit for hair regeneration according to claim 9,
wherein the cell-embedded beads comprises:
mesenchymal cells being hair papilla cells, dermal root sheath cells, skin mesenchymal cells in a developmental period or hair follicle mesenchymal cells induced from pluripotent cells; and
a biocompatible hydrogel of 1.0 mg/mL or more and 3.0 mg/mL or less.

11. The kit for hair regeneration according to Claim 9 or 10,
wherein the biocompatible hydrogel is an extracellular matrix component which gelates.

12. The kit for hair regeneration according to claim 11,
wherein the extracellular matrix component is type I collagen.

13. The kit for hair regeneration according to any of claims 9 to 12,
wherein the cell density of the mesenchymal cells is 5 × 10⁴ cells/cm³ or more and 2 × 10⁵ cells/cm³ or less.

14. Use of the cell-embedded beads according to any one of claims 1 to 5 for hair regeneration.

## Patentansprüche

1. Zelleingebettete Partikel zur Haarregeneration, umfassend:
mesenchymale Zellen und
ein biokompatibles Hydrogel in einer Konzentration von 1,0 mg/ml oder mehr und 10 mg/ml oder weniger,
wobei die mesenchymalen Zellen Haarpapillenzellen, dermale Wurzelscheidenzellen, mesenchymale Zellen der Haut in einer Entwicklungsperiode oder aus pluripotenten Zellen induzierte mesenchymale Haarfollikelzellen sind.

2. Zelleingebettete Partikel zur Haarregeneration nach Anspruch 1, umfassend:
die mesenchymalen Zellen, die Haarpapillenzellen, dermale Wurzelscheidenzellen, mesenchymale Zellen der Haut in einer Entwicklungsperiode oder aus pluripotenten Zellen induzierte mesenchymale Haarfollikelzellen sind, und
ein biokompatibles Hydrogel von 1,0 mg/ml oder mehr und 3,0 mg/ml oder weniger.

3. Zelleingebettete Partikel zur Haarregeneration nach Anspruch 1 oder 2, wobei das biokompatible Hydrogel eine extrazelluläre Matrixkomponente ist, die geliert.

4. Zelleingebettete Partikel zur Haarregeneration nach Anspruch 3, wobei die extrazelluläre Matrixkomponente Typ-I-Kollagen ist.

5. Zelleingebettete Partikel zur Haarregeneration nach einem der Ansprüche 1 bis 4, wobei die Zelldichte der mesenchymalen Zellen 5 × 10⁴ Zellen/cm³ oder mehr und 2 × 10⁵ Zellen/cm³ oder weniger beträgt.

6. Verfahren zur Herstellung von zelleingebetteten Partikeln zur Haarregeneration, umfassend:
Herstellen von Flüssigkeitströpfchen, die mesenchymale Zellen und ein biokompatibles Hydrogel enthalten, und Aushärten des biokompatiblen Hydrogels und
Aggregieren des zelleingebetteten gehärteten Hydrogels zur Suspensionskultivierung unter Verwendung der Zugkraft der Zellen, um die zelleingebetteten Partikel zu erhalten,
wobei die mesenchymalen Zellen Haarpapillenzellen, dermale Wurzelscheidenzellen, mesenchymale Zellen der Haut in einer Entwicklungsperiode oder aus pluripotenten Zellen induzierte mesenchymale Haarfollikelzellen sind und
wobei eine Konzentration des biokompatiblen Hydrogels in den zelleingebetteten Partikeln 1,0 mg/ml oder mehr und 10 mg/ml oder weniger beträgt.

7. Verfahren zur Herstellung von zelleingebetteten Partikeln zur Haarregeneration nach Anspruch 6, wobei die Flüssigkeitströpfchen durch Tropfen auf einen Träger mit einer wasserabweisenden Oberfläche hergestellt werden.

8. Verfahren zur Herstellung von zelleingebetteten Partikeln zur Haarregeneration nach Anspruch 6 oder 7, wobei die Konzentration der in den Flüssigkeitströpfchen enthaltenen mesenchymalen Zellen 5 × 10⁵ Zellen/ml oder mehr und 1 × 10⁸ Zellen/ml oder weniger beträgt.

9. Kit zur Haarregeneration, umfassend:
zelleingebettete Partikel zur Haarregeneration und
Epithelzellen,
wobei die zelleingebetteten Partikel umfassen:
mesenchymale Zellen, die Haarpapillenzellen, dermale Wurzelscheidenzellen, mesenchymale Zellen der Haut in einer Entwicklungsperiode oder aus pluripotenten Zellen induzierte mesenchymale Haarfollikelzellen sind, und
ein biokompatibles Hydrogel von 1,0 mg/ml oder mehr und 10 mg/ml oder weniger.

10. Kit zur Haarregeneration nach Anspruch 9,
wobei die zelleingebetteten Partikel umfassen:
mesenchymale Zellen, die Haarpapillenzellen, dermale Wurzelscheidenzellen, mesenchymale Zellen der Haut in einer Entwicklungsperiode oder aus pluripotenten Zellen induzierte mesenchymale Haarfollikelzellen sind, und
ein biokompatibles Hydrogel von 1,0 mg/ml oder mehr und 3,0 mg/ml oder weniger.

11. Kit zur Haarregeneration nach Anspruch 9 oder 10, wobei das biokompatible Hydrogel eine extrazelluläre Matrixkomponente ist, die geliert.

12. Kit zur Haarregeneration nach Anspruch 11, wobei die extrazelluläre Matrixkomponente Typ-I-Kollagen ist.

13. Kit zur Haarregeneration nach einem der Ansprüche 9 bis 12, wobei die Zelldichte der mesenchymalen Zellen 5 × 10⁴ Zellen/cm³ oder mehr und 2 × 10⁵ Zellen/cm³ oder weniger beträgt.

14. Verwendung der zelleingebetteten Partikel nach einem der Ansprüche 1 bis 5 zur Haarregeneration.

## Revendications

1. Billes enrobées de cellules pour la régénération capillaire comprenant :
des cellules mésenchymateuses ; et
un hydrogel biocompatible à une concentration de 1,0 mg/ml ou plus et de 10 mg/ml ou moins,
dans lesquelles les cellules mésenchymateuses sont des cellules de papille capillaire, des cellules de gaine de racine dermique, des cellules mésenchymateuses de la peau en période de développement ou des cellules mésenchymateuses de follicule pileux induites à partir de cellules pluripotentes.

2. Billes enrobées de cellules pour la régénération capillaire selon la revendication 1, comprenant :
les cellules mésenchymateuses étant des cellules de papille capillaire, des cellules de gaine de racine dermique, des cellules mésenchymateuses de la peau en période de développement ou des cellules mésenchymateuses de follicule pileux induites à partir de cellules pluripotentes ; et
un hydrogel biocompatible de 1,0 mg/ml ou plus et de 3,0 mg/ml ou moins.

3. Billes enrobées de cellules pour la régénération capillaire selon l'une des revendications 1 ou 2,
dans lesquelles l'hydrogel biocompatible est un composant de matrice extracellulaire qui se gélifie.

4. Billes enrobées de cellules pour la régénération capillaire selon la revendication 3,
dans lesquelles le composant de matrice extracellulaire est du collagène de type I.

5. Billes enrobées de cellules pour la régénération capillaire selon l'une quelconque des revendications 1 à 4,
dans lesquelles la densité cellulaire des cellules mésenchymateuses est de 5 x 10⁴ cellules/cm³ ou plus et de 2 x 10⁵ cellules/cm³ ou moins.

6. Procédé pour la production de billes enrobées de cellules pour la régénération capillaire, comprenant :
la préparation de gouttelettes liquides contenant des cellules mésenchymateuses et un hydrogel biocompatible et le durcissement de l'hydrogel biocompatible ; et
l'agrégation de l'hydrogel durci enrobé de cellules pour la culture en suspension en utilisant la force de traction des cellules pour obtenir les billes enrobées de cellules,
dans lequel les cellules mésenchymateuses sont des cellules de papille capillaire, des cellules de gaine de racine dermique, des cellules mésenchymateuses de la peau en période de développement ou des cellules mésenchymateuses de follicule pileux induites à partir de cellules pluripotentes, et
dans lequel une concentration de l'hydrogel biocompatible dans les billes enrobées de cellules est de 1,0 mg/ml ou plus et de 10 mg/ml ou moins.

7. Procédé pour la production de billes enrobées de cellules pour la régénération capillaire selon la revendication 6,
dans lequel les gouttelettes liquides sont préparées en tombant sur un support comportant une surface hydrofuge.

8. Procédé pour la production de billes enrobées de cellules pour la régénération capillaire selon l'une des revendications 6 ou 7,
dans lequel la concentration de cellules mésenchymateuses contenues dans les gouttelettes liquides est de 5 x 10⁵ cellules/ml ou plus et de 1 x 10⁸ cellules/ml ou moins.

9. Kit pour la régénération capillaire, comprenant :
des billes enrobées de cellules pour la régénération capillaire ; et
des cellules épithéliales,
dans lequel les billes enrobées de cellules comprenant :
des cellules mésenchymateuses étant des cellules de papille capillaire, des cellules de gaine de racine dermique, des cellules mésenchymateuses de la peau en période de développement ou des cellules mésenchymateuses de follicule pileux induites à partir de cellules pluripotentes ; et
un hydrogel biocompatible de 1,0 mg/ml ou plus et de 10 mg/ml ou moins.

10. Kit pour la régénération capillaire selon la revendication 9 :
dans lequel les billes enrobées de cellules comprennent :
des cellules mésenchymateuses étant des cellules de papille capillaire, des cellules de gaine de racine dermique, des cellules mésenchymateuses de la peau en période de développement ou des cellules mésenchymateuses de follicule pileux induites à partir de cellules pluripotentes ; et
un hydrogel biocompatible de 1,0 mg/ml ou plus et de 3,0 mg/ml ou moins.

11. Kit pour la régénération capillaire selon l'une des revendications 9 ou 10,
dans lequel l'hydrogel biocompatible est un composant de matrice extracellulaire qui se gélifie.

12. Kit pour la régénération capillaire selon la revendication 11,
dans lequel le composant de matrice extracellulaire est du collagène de type I.

13. Kit pour la régénération capillaire selon l'une quelconque des revendications 9 à 12,
dans lequel la densité cellulaire des cellules mésenchymateuses est de 5 x 10⁴ cellules/cm³ ou plus et de 2 x 10⁵ cellules/cm³ ou moins.

14. Utilisation des billes enrobées de cellules selon l'une quelconque des revendications 1 à 5 pour la régénération capillaire.
